# EUROPEAN PATENT APPLICATION

(11) **EP 4 233 908 A2**
(43) Date of publication of application: **30.08.2023**
(21) Application number: 23169039.7
(22) Date of filing: 13.12.2016
(51) Int. Cl.: A61K 45/00

(54) **NUTRITIONAL COMPOSITIONS AND INFANT FORMULAS TO PROMOTE MYELINATION IN THE BRAIN**

(30) Priority: 14.12.2015 EP 15199758; 30.03.2016 US 201662315312 P; 30.03.2016 US 201662315249 P; 27.04.2016 US 201662328070 P
(62) Divisional of application: 16816630.4
(71) Applicant: Société des Produits Nestlé S.A., 1800 Vevey (CH)
(72) Inventor: SCHNEIDER, Nora, 1066 Epalinges (CH); DEONI, Sean, Providence, 02912 (US); BARTFAI, Tamas, 11328 Stockholm (SE)
(74) Representative: Vossius & Partner Patentanwälte Rechtsanwälte mbB

(57) **Abstract**

The present invention relates to a nutritional composition for infants and young children, such as an infant formula or follow-on formula or growing up milk, preferably an infant formula, and comprising a vitamin or mixtures thereof. The vitamins promotes and/or supports an ideal myelination trajectory in the brain, such trajectory being close to that observed in infants fed exclusively with human breast milk (HBM). The infants or young children can be between 0 and 60 months, preferably between 0 and 12 months of age.

## Description

### Field of the invention

The present invention relates to nutritional compositions for infants and young children and their associated health benefits. In particular, it relates to nutritional compositions comprising a vitamin or mixtures thereof for promoting or supporting an optimal myelination trajectory in the brain, such trajectory being close to that observed in infants fed exclusively with human breast milk (HBM) for the first months of life.

### Background to the Invention

Whenever mothers cannot breast-feed their infants, infant formula provides a suitable alternative to natural feeding with human breast milk. Nutritional compositions for infants and young children are often sold as powders to be reconstituted with water or in some instances as ready to drink or concentrated liquid compositions. These compositions are intended to cover most or all the nutritional needs of the infants or young children.

It is known however, that human breast milk represents the ultimate gold standard in terms of infants' nutrition. Infant formula manufacturers have made many attempts to induce nutritional health effects close to or similar to the benefits of human breast milk.

Mother's milk is recommended for all infants. However, in some cases breast feeding is insufficient or not possible or unsuccessful for medical reasons or the mother chooses not to breast feed. Infant formulae have been developed for these situations.

In many instances however, studies have shown that infant formula do not induce the identical effects on the body compared to human breast milk.

For example, infants fed with infant formula and infants fed with human breast milk (HBM) can exhibit differences in the trajectory of myelination. Myelination normally occurs in the brain starting prenatally and continuing in the offspring postnatally until early adulthood.

Myelination is a fundamental process of neurodevelopment that consist of the ensheathment of the axon by a fat myelin sheath that facilitates action potential transfer. Myelination has been suggested to play a key role in coordinated communication between brain cells and networks. Myelinated white matter matures alongside cognitive and learning abilities.

Infancy and young childhood, especially from the first weeks of life until 2 to 5 year of life, are sensitive periods for brain myelination depending on the brain area.

A recent study ("Breastfeeding and early white matter development: a cross sectional study", Deoni et al, NeuroImage 82, (2013), 77-86) has demonstrated that breast-fed children exhibit increased white matter development in later maturing frontal and association brain regions when compared to infants who are fed with infant formulas. Positive relationships between white matter microstructure and breastfeeding duration are also exhibited in several brain regions that are anatomically consistent with observed improvements in cognitive and behavioural performance measures. The results of this study thus indicate that not only it is important that myelination occurs to avoid cognitive deficits but also that this happens early in the infant's life, ideally following the trajectory (myelination over time) that would be provided in an infant who is breast fed. Additionally, the study indicates that this optimal myelination trajectory is normally not provided by standard infant formulas.

Accordingly, there is a need to provide a nutritional composition for infants or young children which would promote and/or support an optimal myelination trajectory in the brain, such trajectory being close to that observed in infants fed exclusively with human breast milk (HBM).

There is a need, for infants fed with infant formula, to promote and/or support an optimal myelination trajectory in the brain, such trajectory being close to that observed in infants fed exclusively with human breast milk (HBM).

There is also a need, for infants fed with infant formula, to provide them with the best nutrition that enables promoting and/or supporting an optimal myelination trajectory in the brain, such trajectory being close to that observed in infants fed exclusively with human breast milk (HBM).

There is a need, for infants fed with infant formula, to promote and/or support an optimal short term or long term health status through a nutrition promoting and/or supporting an optimal myelination trajectory in the brain, such trajectory being close to that observed in infants fed exclusively with human breast milk (HBM); such health status including an optimal brain and cognitive functions development as well as prevention of neurocognitive deficits.

There is a need to compensate for the sub-optimal myelination observed in non-breast-fed infants.

### Summary of the invention

The invention relates to a nutritional composition for infants and young children, such as a pre-term formula, an infant formula, follow-on formula,growing up milk or baby food, preferably an infant formula. The composition comprises a vitamin or mixtures thereof. A vitamin or mixtures thereof promotes or supports an optimal myelination trajectory in the brain, such trajectory being close to that observed in infants fed exclusively with human breast milk (HBM).

In one aspect, a nutritional composition for infants and/or young children is provided comprising a vitamin or mixtures thereof for promoting and/or supporting an optimal myelination trajectory in the brain, such trajectory being close to that observed in infants fed exclusively with human breast milk (HBM).

In one aspect, a nutritional composition for infants and/or young children is provided comprising a vitamin or mixtures thereof for promoting and/or supporting an optimal myelination trajectory in the brain, such trajectory being close to that observed in infants fed exclusively with human breast milk (HBM), which results in promoting and/or supporting an health status characterized by optimal brain and cognitive functions' development and/or prevention of neurocognitive deficits.

In another aspect, the use of a vitamin or mixtures thereof is provided in the manufacture of a nutritional composition to be administered to infants and/or young children to promote and/or support an optimal myelination trajectory in the brain, such trajectory being close to that observed in infants fed exclusively with human breast milk (HBM).

In another aspect, the use of a vitamin or mixtures thereof is provided in the manufacture of a nutritional composition to be administered to infants and/or young children to promote and/or support an optimal myelination trajectory in the brain, such trajectory being close to that observed in infants fed exclusively with human breast milk (HBM), such trajectory resulting in promoting and/or supporting an health status characterized by optimal brain and cognitive functions' development and/or prevention of neurocognitive deficits.

In a further aspect, a method of promoting and/or supporting an optimal myelination trajectory in the brain, such trajectory being close to that observed in infants fed exclusively with human breast milk (HBM), is provided comprising administering to an infant and/or young children nutritional composition comprising a vitamin or mixtures thereof.

In a further aspect, a method of promoting and/or supporting an optimal myelination trajectory in the brain is provided comprising administering to infants and/or young children nutritional composition comprising a vitamin or mixtures thereof , such trajectory being close to that observed in infants fed exclusively with human breast milk (HBM) and resulting in promoting and/or supporting an health status characterized by optimal brain and cognitive functions' development and/or prevention of neurocognitive deficits.

In another aspect, the use of a vitamin or mixtures thereof is provided to promote and/or support an optimal myelination trajectory in the brain, such trajectory being close to that observed in infants fed exclusively with human breast milk (HBM), in an infant and/or young children.

In another aspect, the use of a vitamin or mixtures thereof is provided to promote and/or support an optimal myelination trajectory in the brain in infants and/or young children, such trajectory being close to that observed in infants fed exclusively with human breast milk (HBM) and resulting in promoting and/or supporting an health status characterized by optimal brain and cognitive functions' development and/or prevention of neurocognitive deficits.

The infants or young children can be between 0 and 60 months, or between 0 and 24 months of age, or between 0 and 12 months of age, or between 0 and 6 months of age. In an additional aspect, a nutritional composition is provided which comprises in addition to a vitamin (for example folic acid and/or Vitamin B12) or mixtures thereof: minerals (in particular iron, Magnesium, phosphorus, copper, calcium and/or zinc), a phospholipid (for example sphingomyelin) a phospholipid, a metabolic precursor and/or a metabolite thereof, a fatty acid derivative (for example DHA and/or ARA, nervonic acid and/or stearic acid), and/or choline.

In a further aspect of the invention, a nutritional composition is provided which comprises sphingomyelin, iron, choline, DHA and folic acid.

### Brief Description of the Figures

**Figures** 1: shows myelination trajectories in infants and young children breastfed vs fed with two commercial formulas comprising different levels of folic acid. Data are obtained from the experiment described in Example 2.
**Figure 2****:** shows the structure of 2-amino-4-octadecene-1,3-diol (sphingosine as below defined).
**Figure 3****:** shows the structure of quaternary ammonium salts containing the *N,N,N-*trimethylethanolammonium cation (Choline as below defined).
Figure 4: Shows the impact of DHA on MBP, NF, and/or MBP/NF at day 18 and/or day 30.
Figure 5: Shows the impact of stearic acid on A2B5, MBP, MAG, NF, MBP/NF, and/or MAG/NF at day 6, day 18 and/or day 30.
Figure 7: Shows the impact of vitamin B12 on A2B5, NF, MBP/NF, and/or MAG at day 12, day 18 and/or day 30.
Figure 8: Shows the impact of folic acid on A2B5, NF, MAG, MAG/NF, and/or MBP/NF at day 12, day 18 and/or day 30.
Figure 9: Shows the impact of choline on A2B5, MAG and/or MBP at day 12, day 18 or day 30.
Figure 13: Shows the impact of Iron on A2B5, MBP, MAG, NF, and/or MAG/NF at day 12, day 18 and/or day 30.

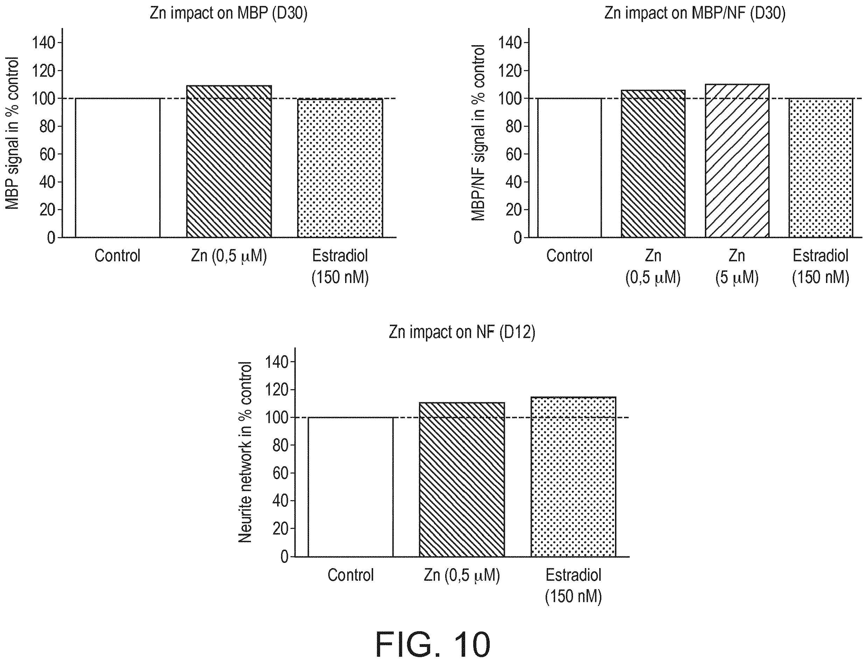

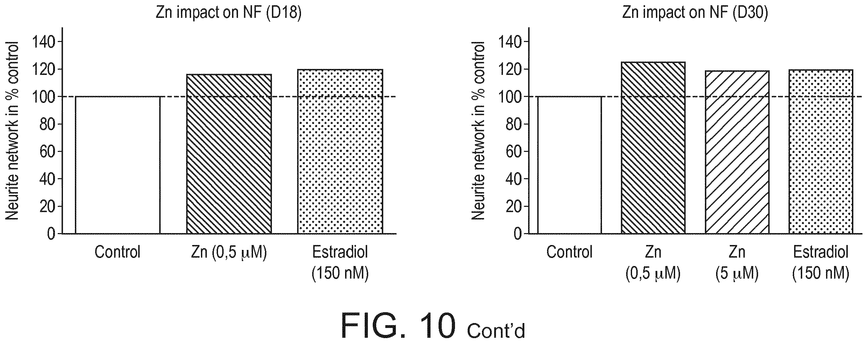

Figure 10: Shows the impact of Zinc on MBP, NF and/or MBP/NF at day 12, day 18 and/or day 30.

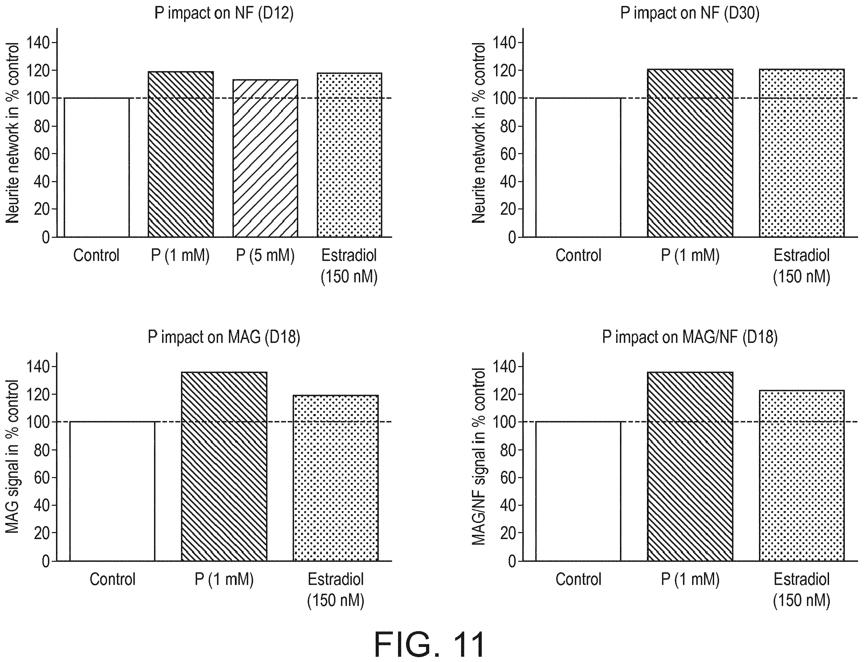

Figure 11: Shows the impact of phosphorus on MAG, NF, and/or MAG/NF at day 12, day 18 and/or day 30.

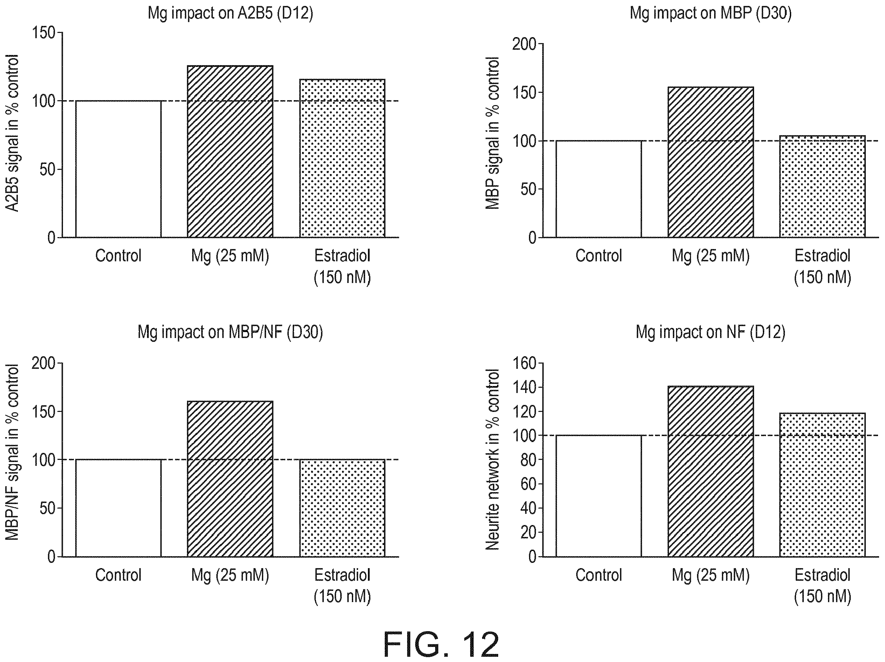

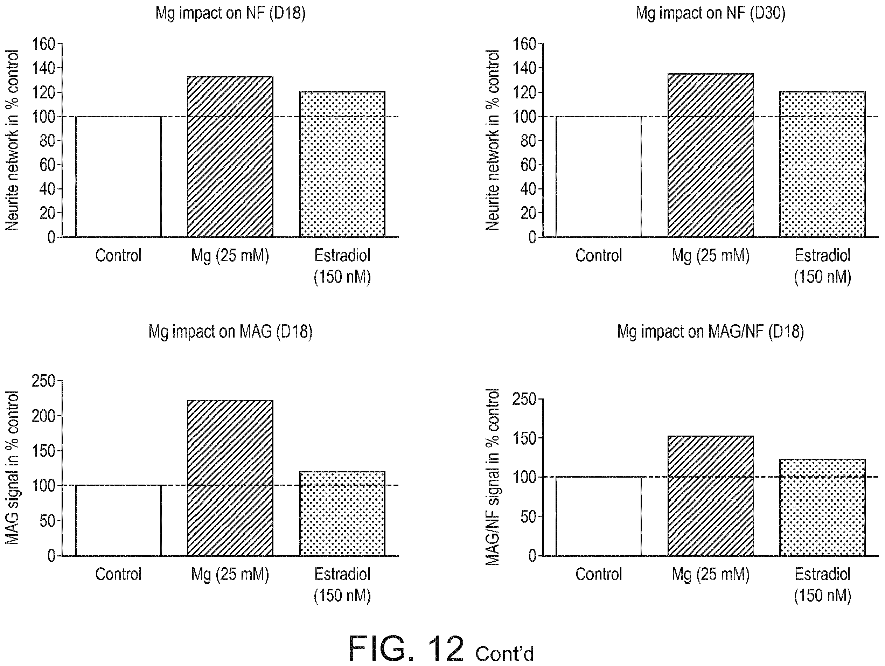

Figure 12: Shows the impact of magnesium on A2B5, MBP, NF, MAG, MBP/NF and/or MAG/NF at day 12, day 18 and/or day 30.

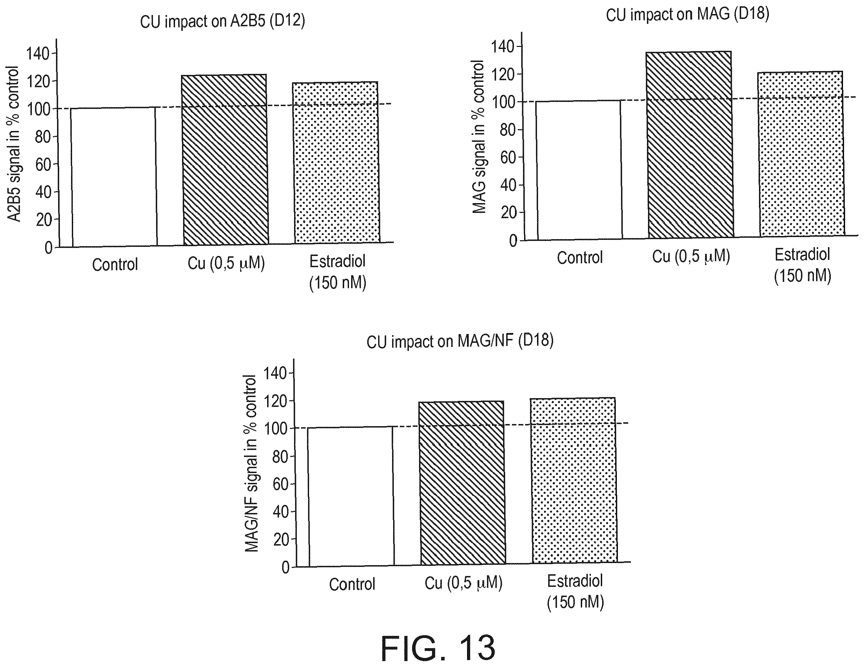

Figure 13: Shows the impact of copper on A2BF, MAG, and/or MAG/NF at day 12 and/or day 18.

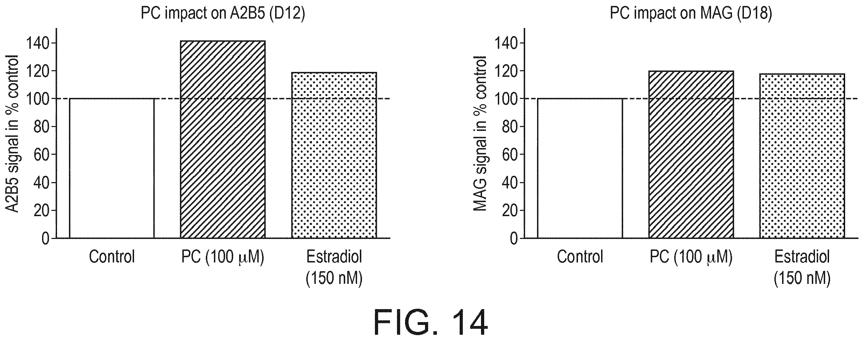

Figure 14: shows the impact of phosphatidylcholine on A2B5 at day 12 and on MAG at day 18.

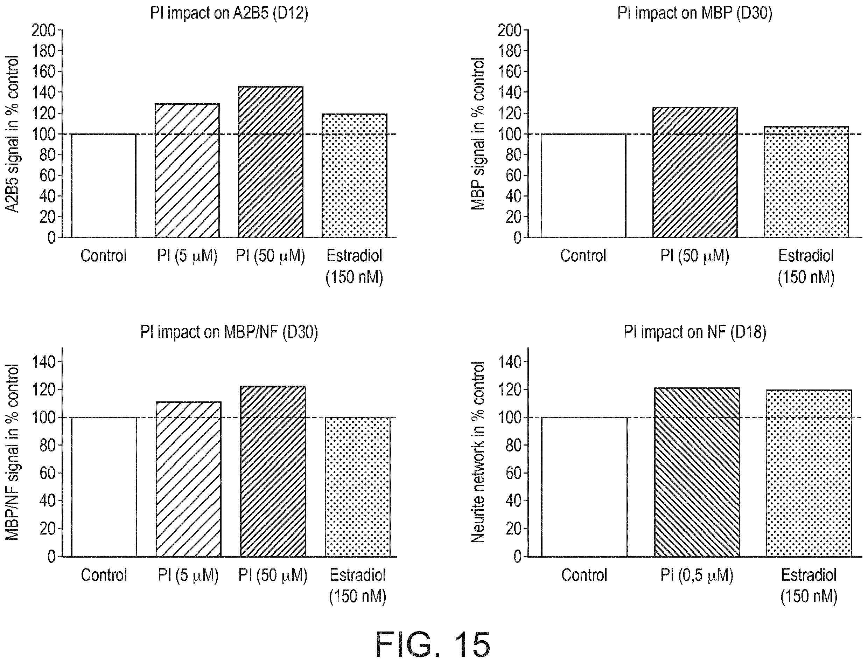

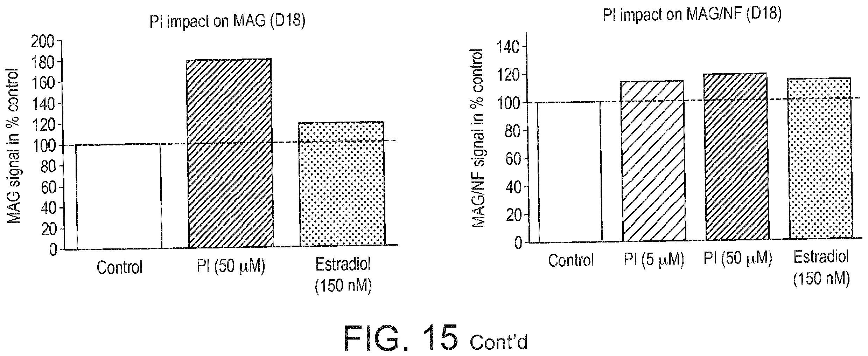

Figure 15: Shows the impact of phosphatidylinositol on A2B5, MBP, MAG, NF, MAG/NF at day 12, day 18 and/or day 30.

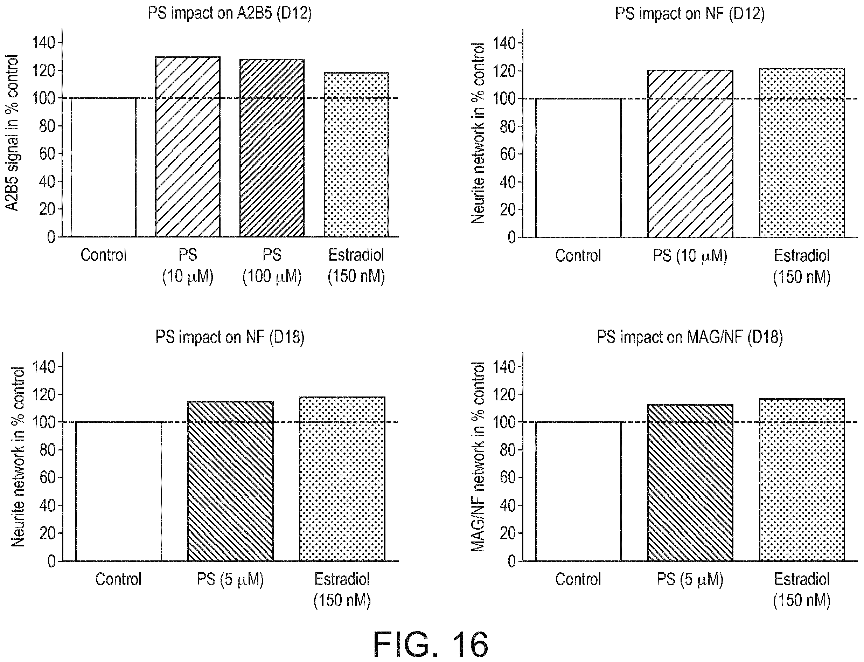

Figure 16: Shows the impact of phosphatidylserine on A2B5, NF, and/or MAG/NF at day 12 and/or D18.

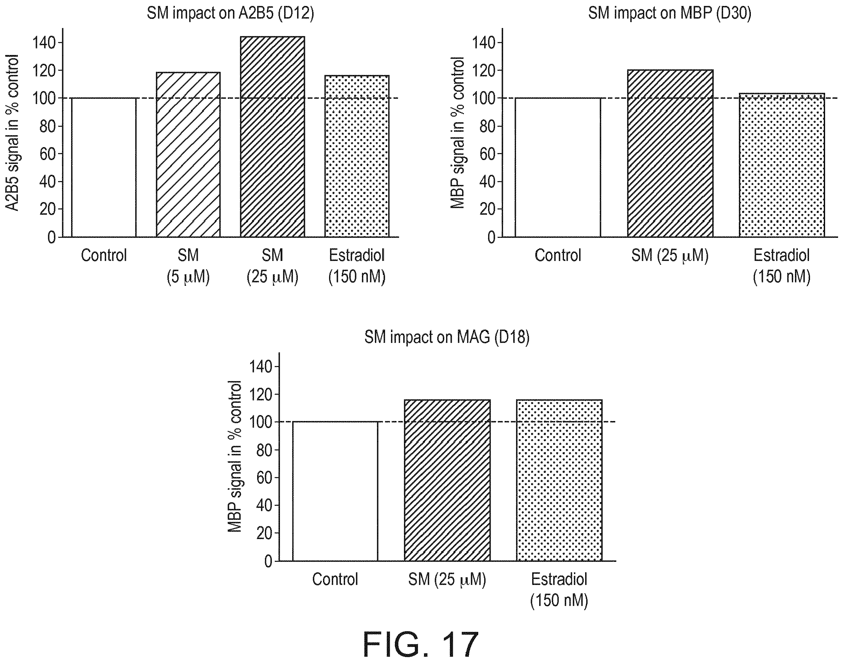

Figure 17: Shows the impact of sphingomyelin on A2B5, MAG, and/or MBP at day 12, day 18 and/or day 30.

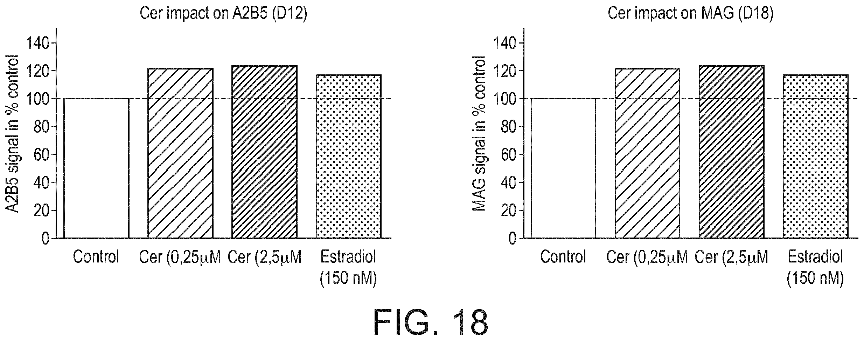

Figure 18: Shows the impact of ceramide on A2B5 at day 12, and on MAG at day 18.

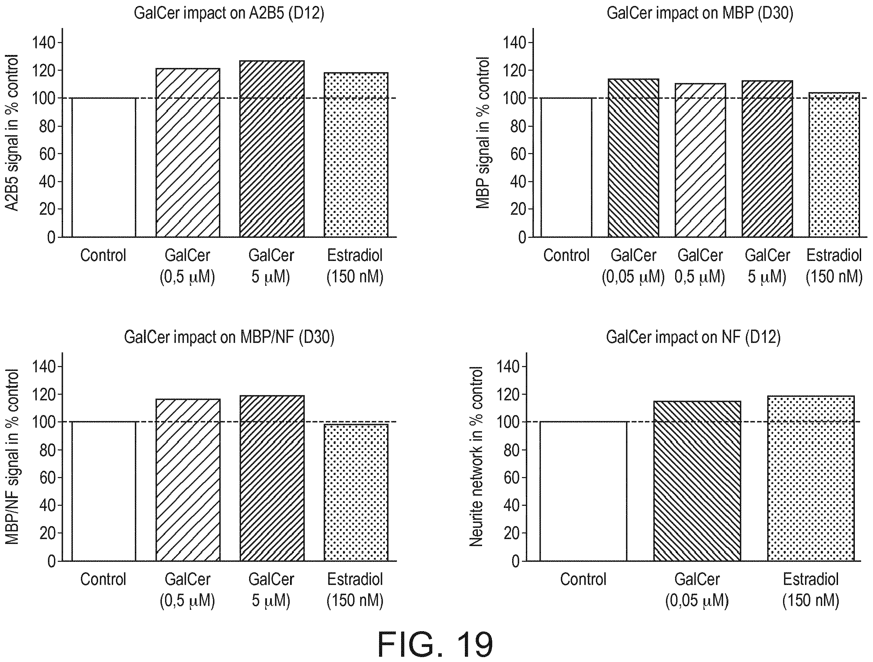

Figure 19: Shows the impact of galactoceramide on A2B5, MBP, NF, and/or MBP/NF at day 12 and/or day 30.

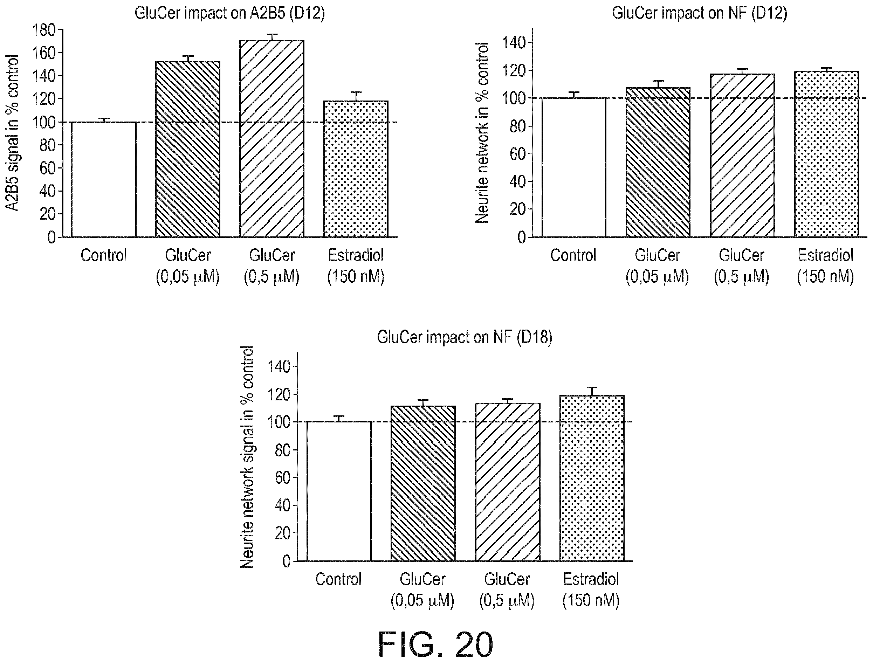

Figure 20: Shows the impact of glucoceramide on A2B5 at day 12 and NF at day 12 and day 18.

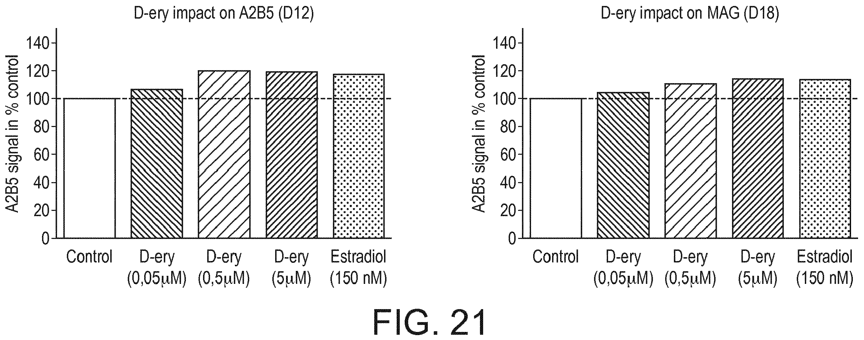

Figure 21: Shows the impact of D-erythroceramide on A2B5 at day 12 and on MAG at day 18.

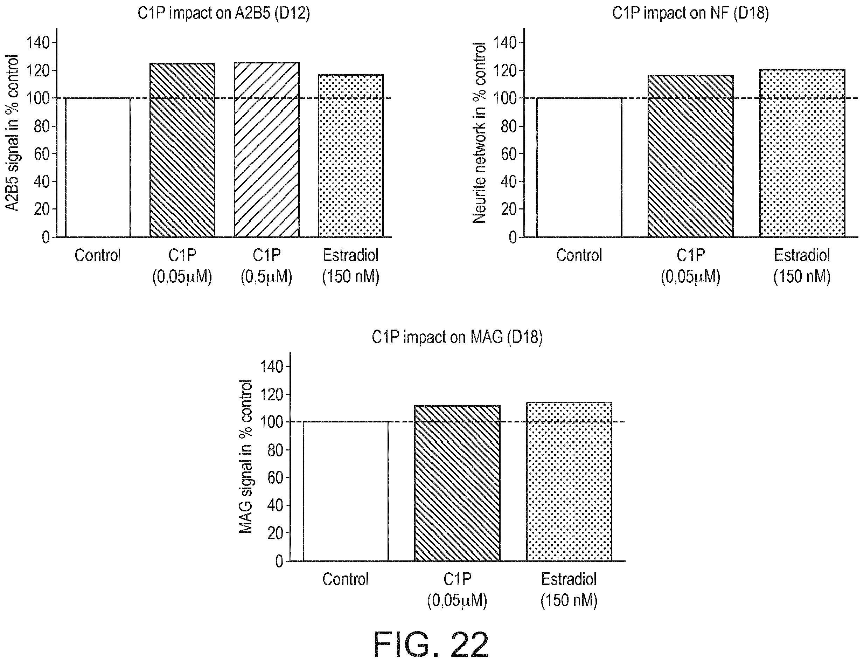

Figure 22: Shows the impact of Ceramide-1-phosphate on A2B5 at day 12, and on NF and MAG at day 18.

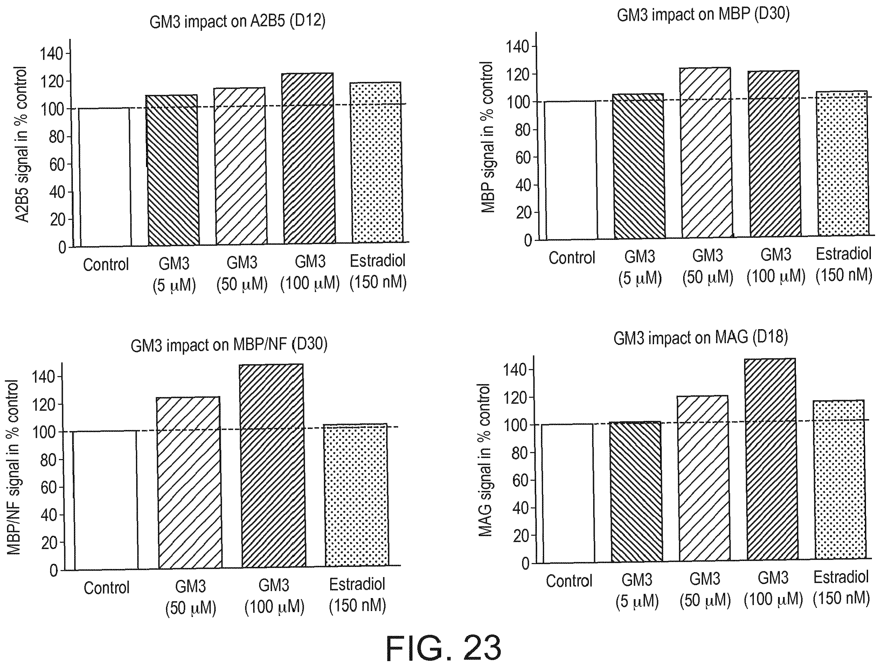

Figure 23: Shows the impact of monosialoganglioside-3 (GM3) on A2B5, MBP, MAG, and/or MBP/NF at day 12, day 18 and/or day 30.

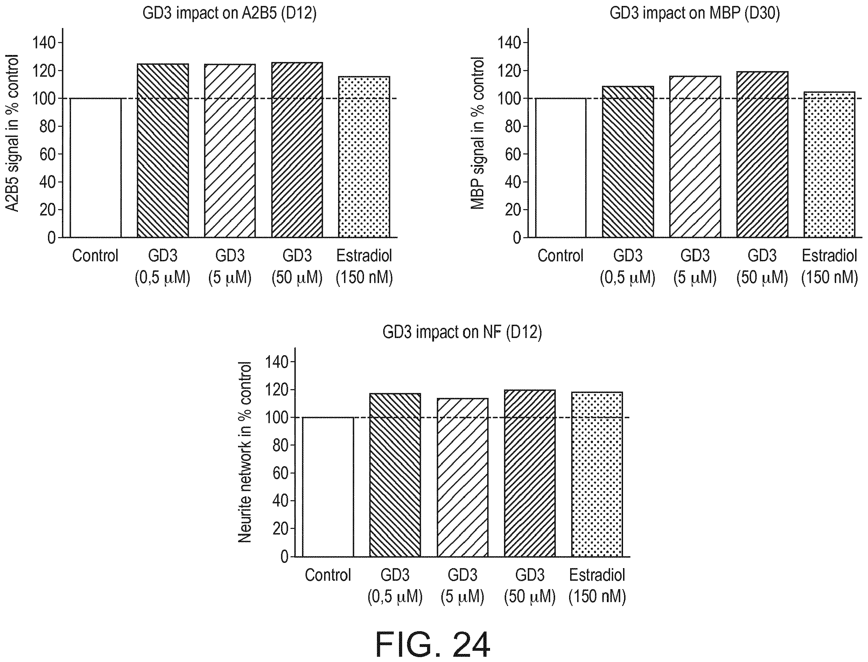

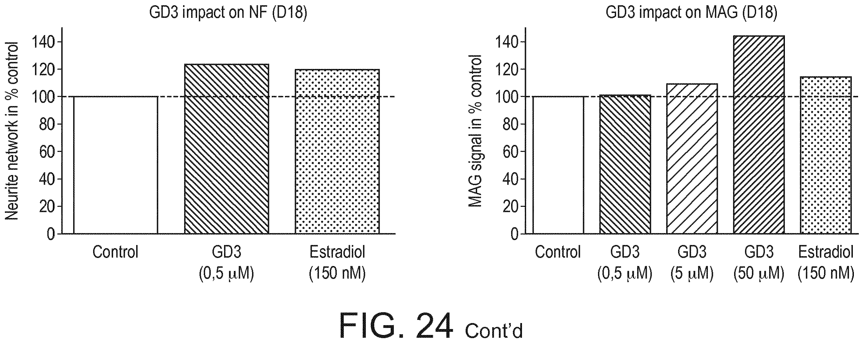

Figure 24: Shows the impact of disialogangliosides 3 (GD3) on A2B5, MBP, NF and/or MAG at day 12, day 18 and/or day 30.

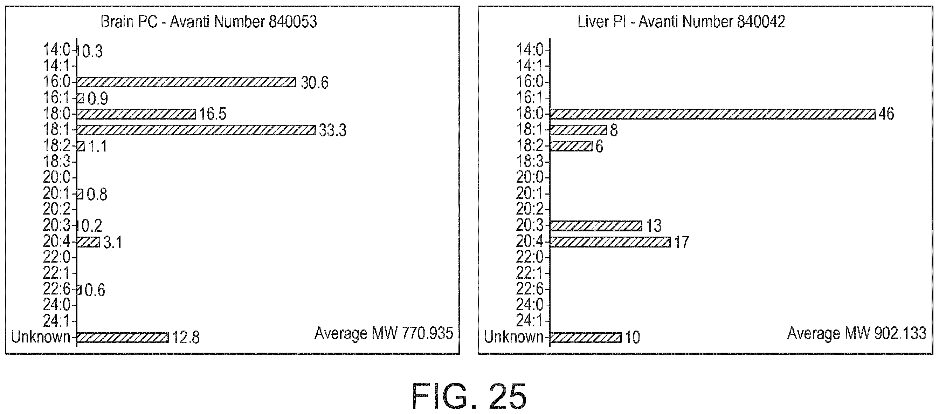

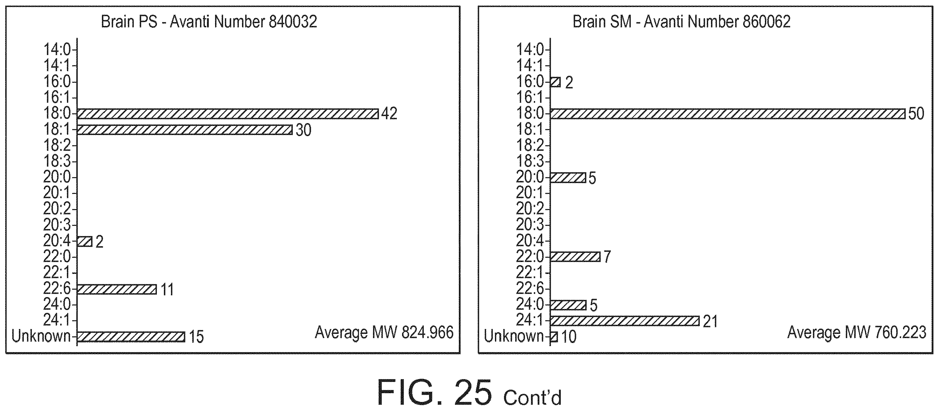

Figure 25: Shows the fatty acid profile of Phosphatidylinositol (PI), Phosphatidylcholine, Phosphatidyl (PC), Phosphatidylserine (PS), and Sphingomyelin used in example 3.

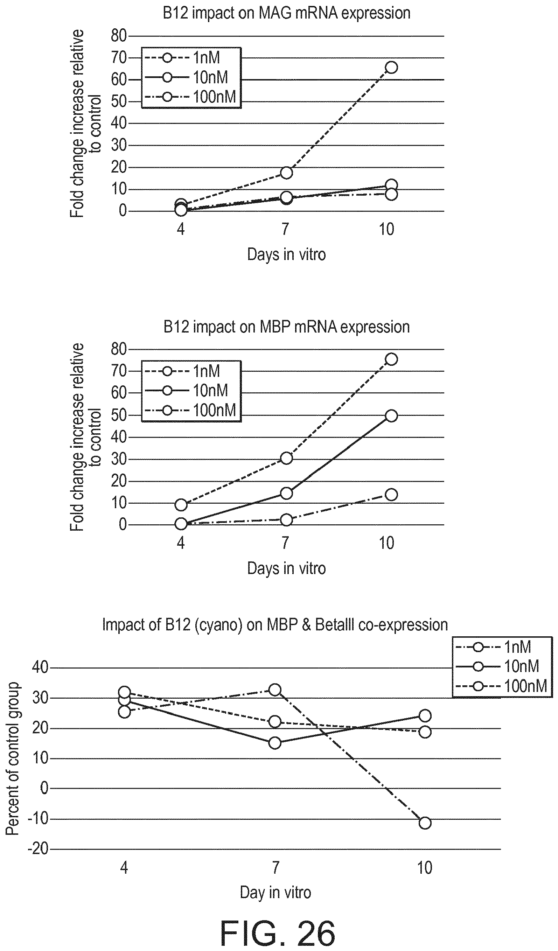

Figure 26: Shows the impact of vitamin B12 on MAG and MBP mRNA expression and on MBP and Betalll Co-expression.

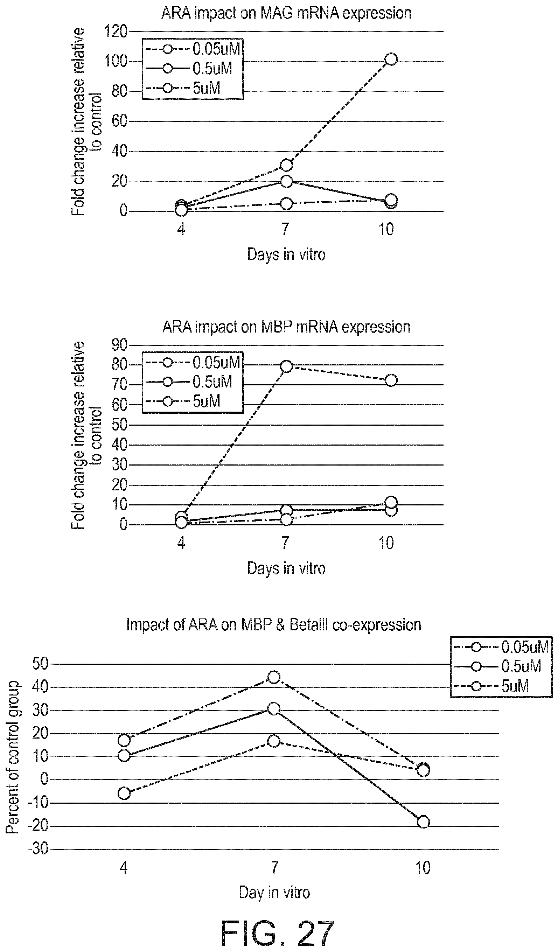

Figure 27: Shows the impact of ARA on MAG and MBP mRNA expression and on MBP and Betalll Co-expression.

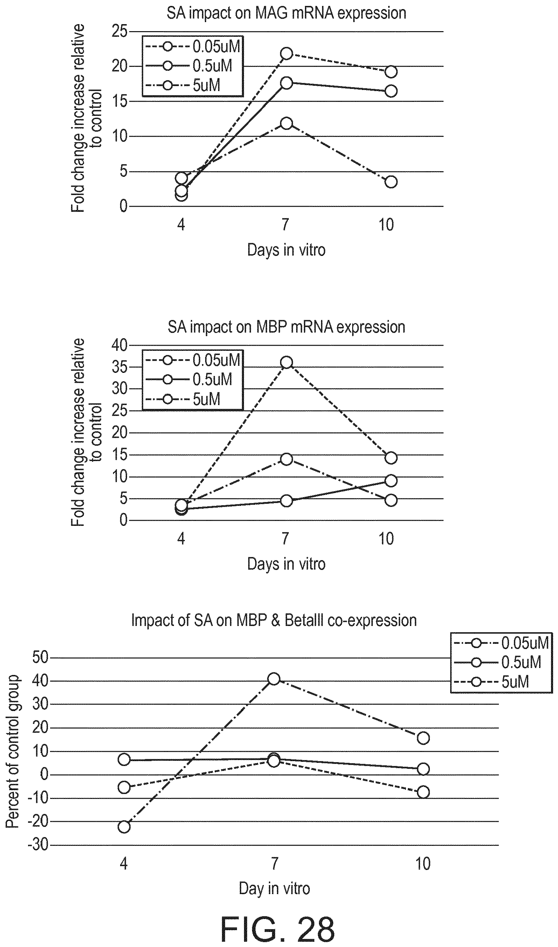

Figure 28: Shows the impact of stearic acid on MAG and MBP mRNA expression and on MBP and Betalll Co-expression.

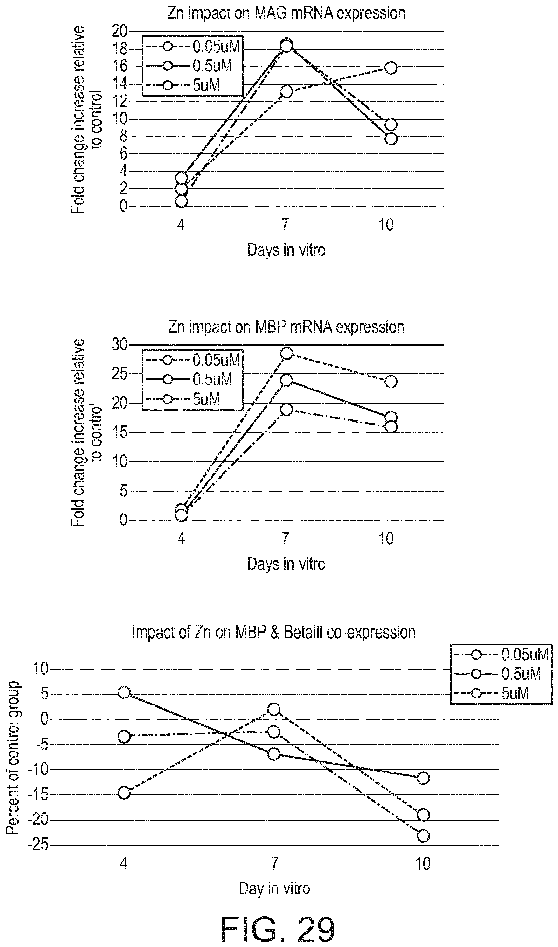

Figure 29: Shows the impact of zinc on MAG and MBP mRNA expression and on MBP and Betalll Co-expression.

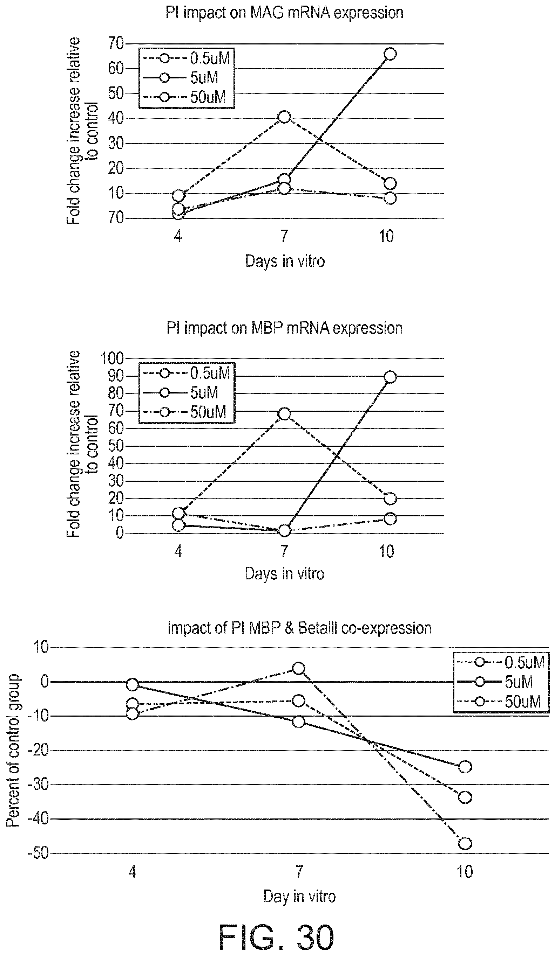

Figure 30: Shows the impact of phosphatidylinositol on MAG and MBP mRNA expression.

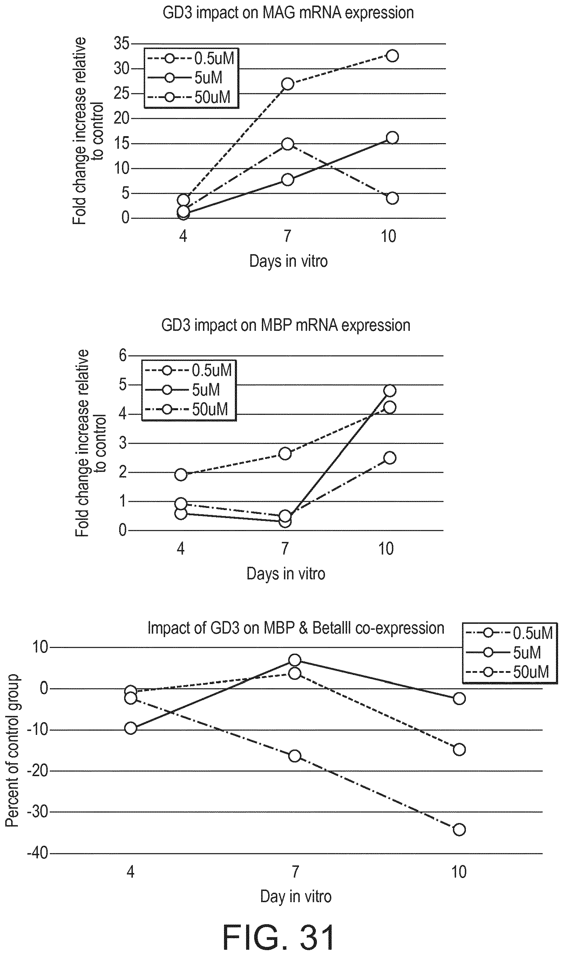

Figure 31: Shows the impact of GD3 on MAG and MBP mRNA expression and on MBP and Betalll Co-expression.

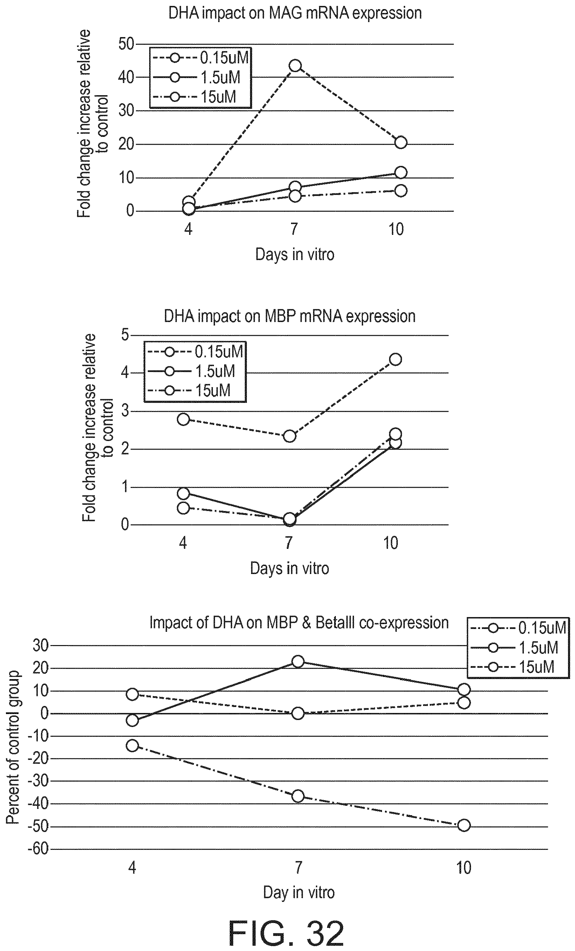

Figure 32: Shows the impact of DHA on MAG and MBP mRNA expression and on MBP and Betalll Co-expression.

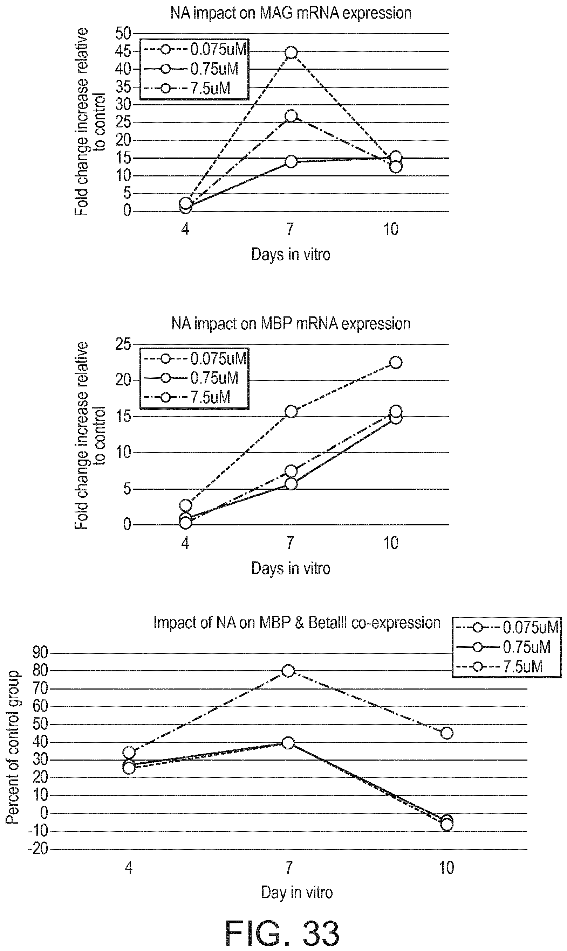

Figure 33: Shows the impact of nervonic acid on MAG and MBP mRNA expression and on MBP and Betalll Co-expression.

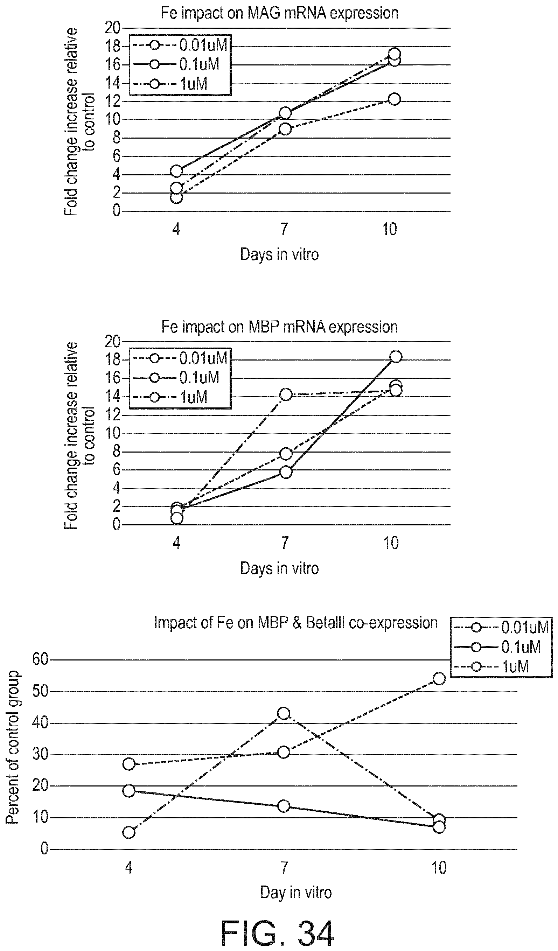

Figure 34: Shows the impact of Iron on MAG and MBP mRNA expression and on MBP and Betalll Co-expression.

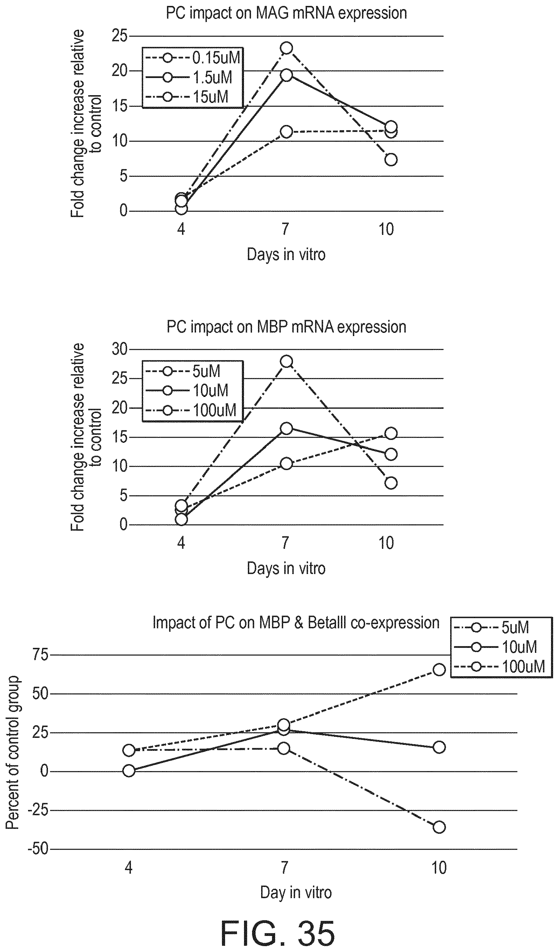

Figure 35: Shows the impact of phosphatidylcholine on MAG and MBP mRNA expression and on MBP and Betalll Co-expression.

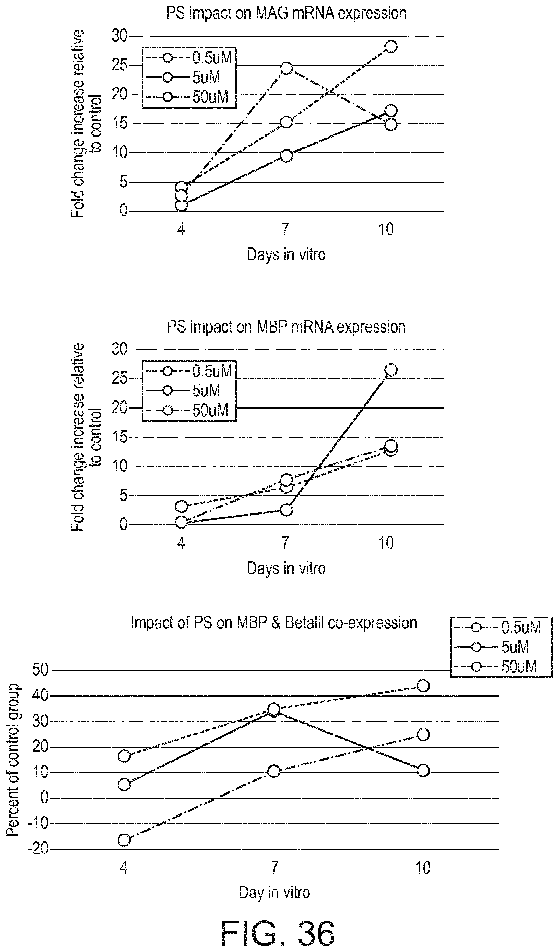

Figure 36: Shows the impact of phosphatidylserine on MAG and MBP mRNA expression and on MBP and Betalll Co-expression.

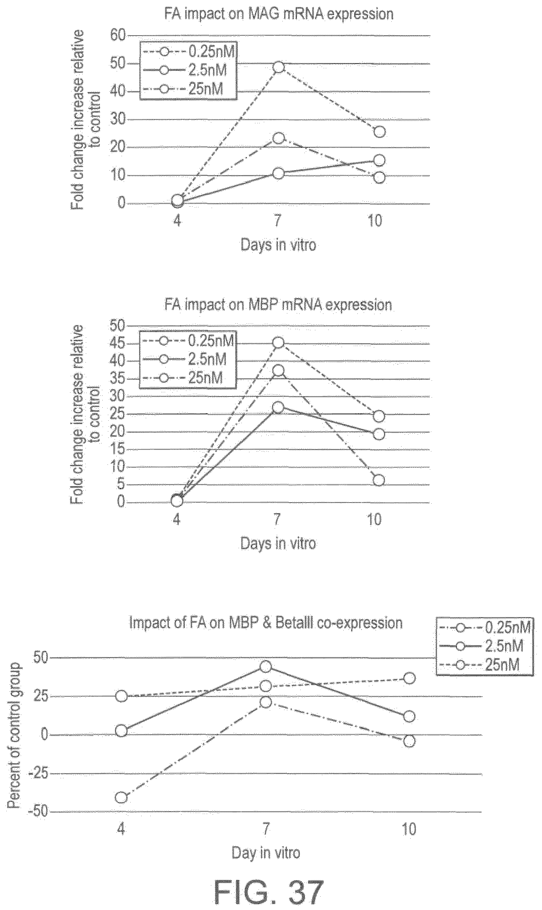

Figure 37: Shows the impact of folic acid on MAG and MBP mRNA expression and on MBP and Betalll Co-expression.

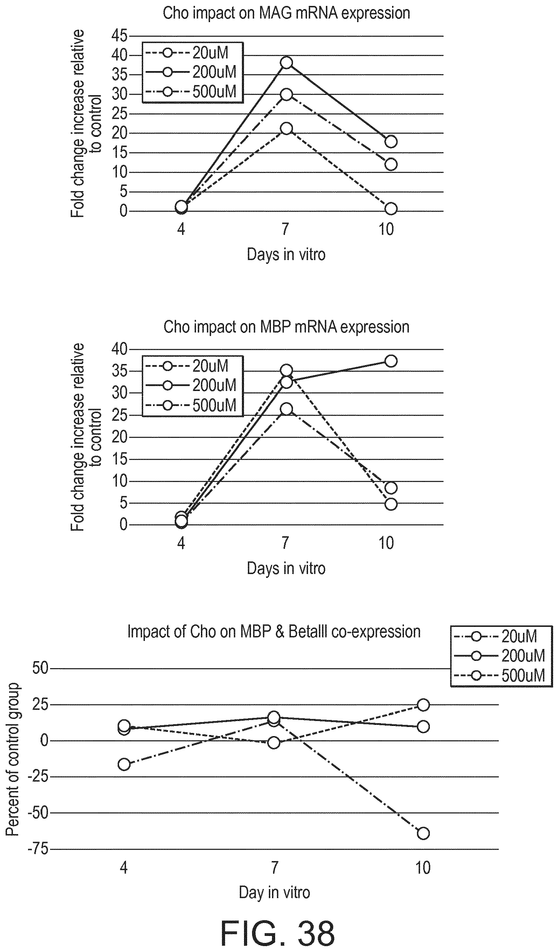

Figure 38: Shows the impact of choline on MAG and MBP mRNA expression and on MBP and Betalll Co-expression.

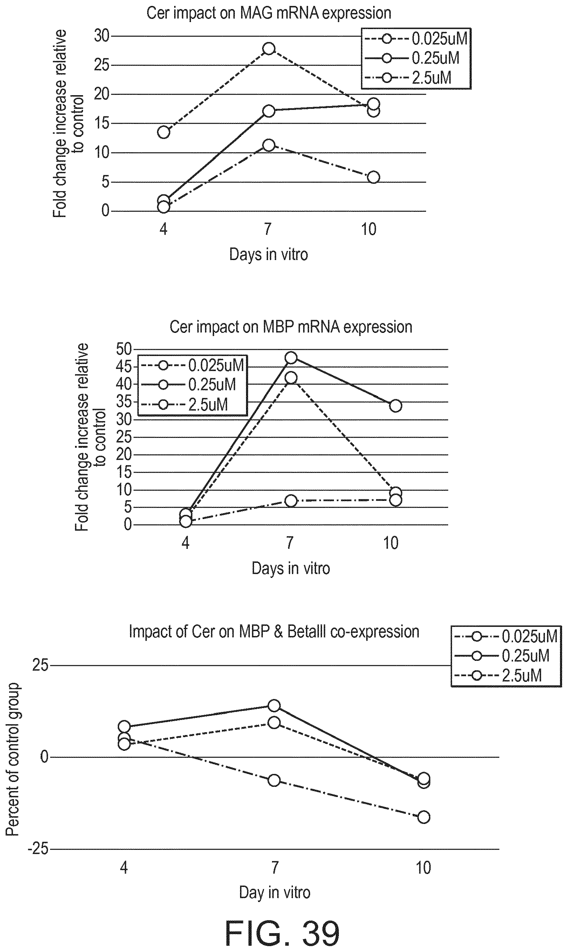

Figure 39: Shows the impact of ceramide on MAG and MBP mRNA expression and on MBP and Betalll Co-expression.

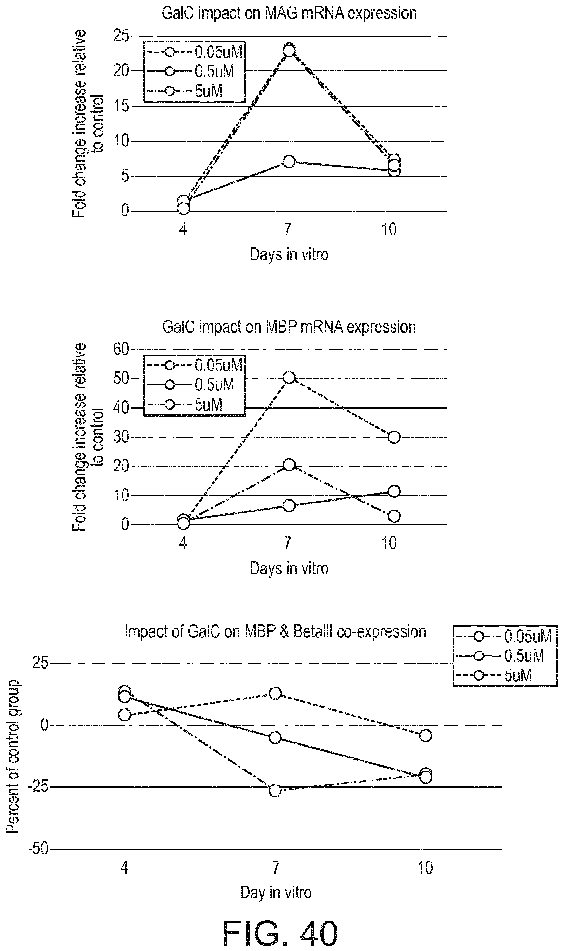

Figure 40: Shows the impact of galactoceramide on MAG and MBP mRNA expression and on MBP and Betalll Co-expression.

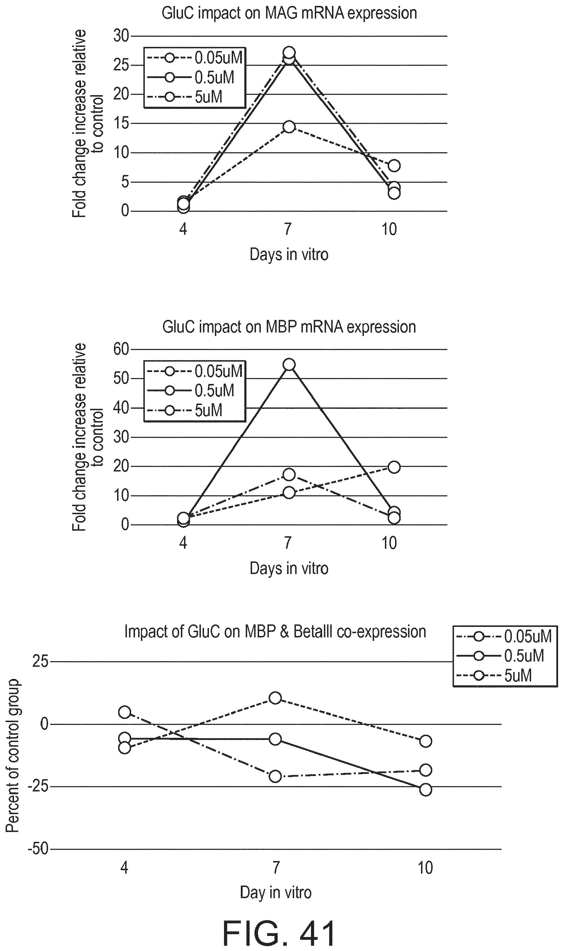

Figure 41: Shows the impact of glucoceramide on MAG and MBP mRNA expression and on MBP and Betalll Co-expression.

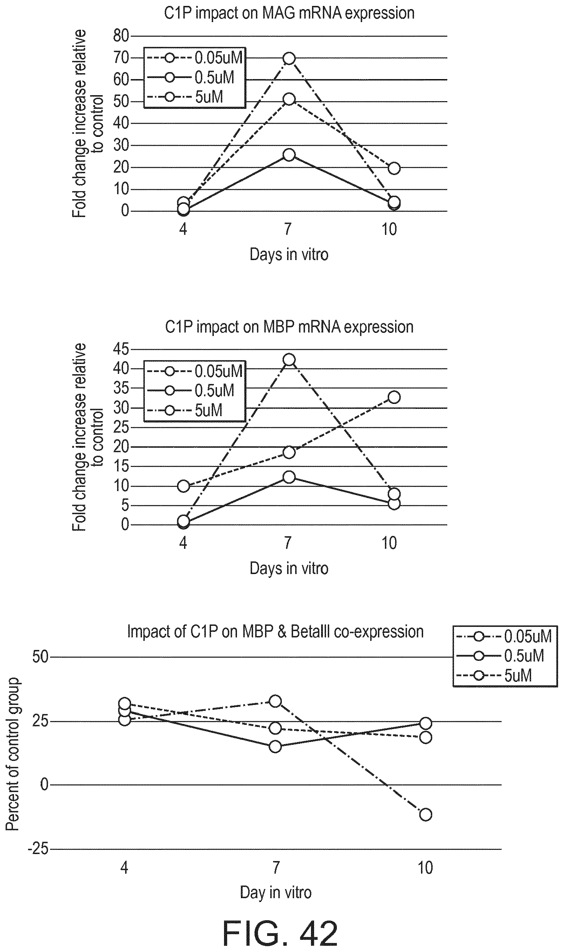

Figure 42: Shows the impact of Ceramide-1-phosphate on MAG and MBP mRNA expression and on MBP and Betalll Co-expression.

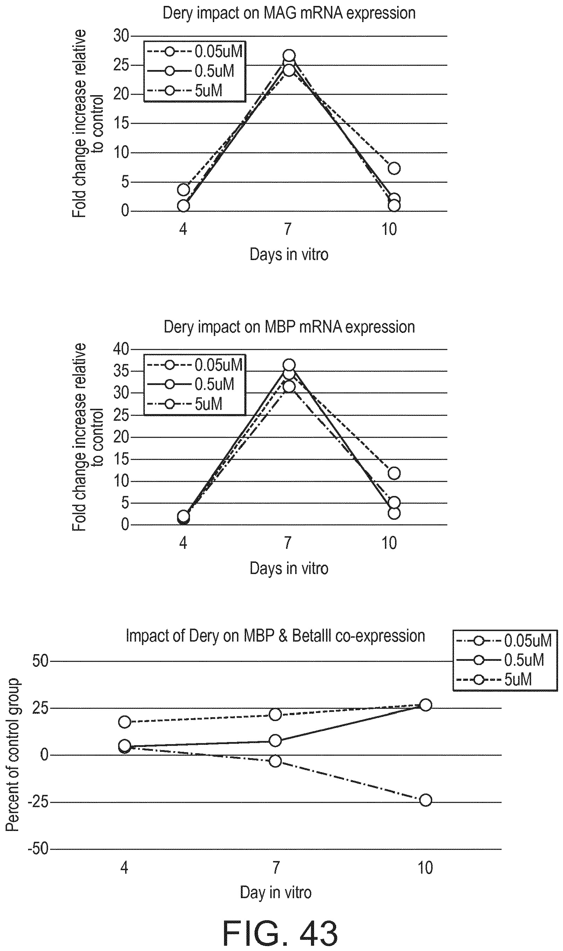

Figure 43: Shows the impact of D-erythroceramide on MAG and MBP mRNA expression and on MBP and Betalll Co-expression.

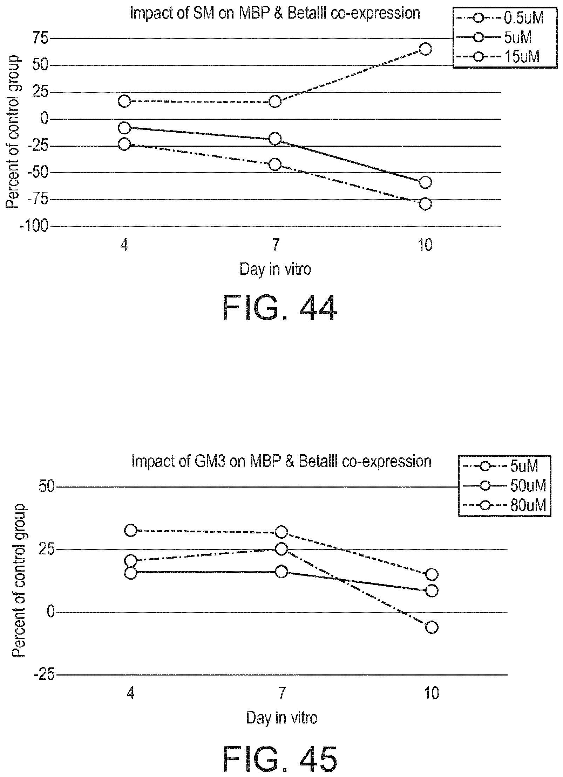

Figure 44: Shows the impact of sphingomyelin on MBP and Betalll Co-expression.
Figure 45: Shows the impact of GM3 on MBP and Betalll Co-expression.

### Description of the invention

### Definitions

As used herein, the following terms have the following meanings.

Within the context of the present invention, the term "trajectory of myelination" indicates the extent of myelination (as linked to Myelin Water Fraction) as a function of time across infancy and early childhood.

Within the context of the present invention, the term "optimal trajectory of myelination" indicates a myelination trajectory as above defined which is as close as possible to that achieved in infants which are exclusively breast-fed during their first (3) months of life.

An infant's myelination trajectory may be considered to be as close as possible to that achieved in infants which are exclusively breastfed during their first (3) months of life, if the distance between any equivalent/same measurement points on the infant's trajectory and said exclusively breastfed infant's trajectory is up to 50%, in particular up to 25%, more particularly up to 20%. Non limiting examples within the range of up to 50% include, 50%, 40%, 30%, 25%, 20%, 10%, 5%, 1%, 0.5%, and 0.01%. In particular the trajectories will be considered bioequivalent.

The myelination trajectory can be measured at any combination of time points. In particular the time points are within the first 5 years of a human's life, more particularly the first 2 & 3 years of a human's life, even more particularly in the first year of a human's life.

The myelination trajectory may be determined by measuring the myelin associated water fraction and/or the myelin associated water pool in a subject at different times points, in particular at different time points across the first 5 years of a human subject's life, more particularly the first 2 & 3 years of a human's life, even more particularly the 1st year of a human's life. The myelin associated water fraction and/or the myelin associated water pool in a subject may be measured using a multicomponent relaxation (MCR) magnetic resonance imaging (MRI) technique and in particular using the mcDESPOT technique (Deoni et al 2008). In particular the myelination trajectory may be determined by measuring the myelin associated water pool using the mcDESPOT technique (Magn.Reson.Med.2008 60:1372-1387 the subject matter of which is hereby incorporated by reference).

Within the context of the present invention, the term "promote" and/or "promoting" indicates a factor or a number of factors causing a certain process to occur.

Within the context of the present invention, the term "support" and/or "supporting" indicates a factor or a number of factors sustaining a certain process once it has started to occur.

Within the context of the present invention, the expression "Learning" refers to the acquisition of knowledge or skills through experience, study, or by being taught.

The term "cogniton" refers to an intellectual process by which one individual becomes aware of, perceives, or comprehends ideas; thus, the ability to think and understand. Cognition includes all aspects of information processing, perception, attention, thinking, reasoning, understanding and remembering as well as psychomotor, language, memory, concentration, executive functions and problem-solving abilities.

The term "infant" means a child under the age of 12 months.

The expression "young child" means a child aged between one and five years, (including toddlers).

The expression "child" generally indicates a human up to the age of eighteen.

A "preterm" or "premature" means an infant or young child that was not born at term. Generally it refers to an infant born prior to the completion of 37 weeks of gestation.

The expression "Term born infant" indicates an infant born after 37 weeks gestation.

Within the context of the present invention, the term "Low birth weight" indicates a newborn's body weight below 2500g (5.5 pounds), either as a result of preterm birth (i.e. before 37 weeks of gestation) and/or due to restricted foetal growth.

Within the context of the present invention, the term "Small-for-gestational-age (SGA)" refers to babies with birth weights below the 10th percentile for babies of the same gestational age.

The expression "Postnatal period" is the period beginning immediately after the birth of a child and extending for about six weeks.

The expression "nutritional composition" means a composition which nourishes a subject. This nutritional composition is usually to be taken enterally, orally, parenterally or intravenously, and it usually includes a lipid or fat source and a protein source. Preferably, a nutritional composition is for oral use.

The expression "hypoallergenic nutritional composition" means a nutritional composition which is unlikely to cause allergic reactions.

The expression "synthetic composition" means a mixture obtained by chemical and/or biological means, which can be chemically identical to the mixture naturally occurring in mammalian milks.

The expression "infant formula" means a foodstuff intended for particular nutritional use by infants during the first four to six months of life and satisfying by itself the nutritional requirements of this category of person (Article 1.2 of the European Commission Directive 91/321/EEC of May 14, 1991 on infant formulae and follow-on formulae).

The expression "starter infant formula" means a foodstuff intended for particular nutritional use by infants during the first four months of life.

The expression "follow-on formula" means a foodstuff intended for particular nutritional use by infants aged over four months and constituting the principal liquid element in the progressively diversified diet of this category of person.

Within the context of the present invention, the term "Growing up milk (GUM)" indicates nutritional formula which may be given to children after stopping the infant formula. The "growing-up milks" (or GUMs) are given from one year onwards. It is generally a milk-based beverage adapted for the specific nutritional needs of young children.

The expression "baby food" means a foodstuff intended for particular nutritional use by infants during the first years of life.

The expression "fortifier" refers to liquid or solid nutritional compositions suitable for mixing with breast milk or infant formula.

The term "weaning period" means the period during which the mother's milk is substituted by other food in the diet of an infant.

The "mother's milk" should be understood as the breast milk or colostrum of the mother (= Human Breast Milk = HBM).

The term "oligofructose" as used herein refers to a fructose oligomers. It can be long chain or short chain , pending on the degree of polymerization of the oligofructose (number of monomers). Preferably the oligofructose of the invention is a short-chain oligofructose, most preferably it has a degree of polymerization of from 2 to 10, for example a degree of polymerization of from 2 to 8.

The term "*sn*-2 palmitate" as used herein refers to palmitic acid in the *sn*-2 position of the triglyceride to which it is bonded.

"High sn-2 palmitate triglyceride" refers to a triglyceride (TG) containing more than 30% of the palmitic acids in the sn-2 position. For example a commercially available high sn-2 palmitate ingredient is sold by Lipid Nutrition is Betapol^{™} B-55. It is a triglyceride mixture derived from vegetable oil in which at least 54% of the palmitic acid is in the *sn*-2 position of the glycerol molecule.

"Alpha-Lactalbumin" refers to a high-quality, easy-to-digest whey protein that comprises 20-25% of total human breast milk (HBM) protein and is the primary protein found in HBM . The structure of alpha-lactalbumin is comprised of 123 amino acids and 4 disulfide bridges and the protein has a molecular weight of 14.2K Daltons. Alpha-lactalbumin is ideal for lower protein infant formulas due to its high content of essential amino acids, particularly tryptophan. Alpha-lactalbumin also represents a source of sphingomyelin according to the present invention.

The term "prebiotic" means non-digestible carbohydrates that beneficially affect the host by selectively stimulating the growth and/or the activity of healthy bacteria such as bifidobacteria in the colon of humans (Gibson GR, Roberfroid MB. Dietary modulation of the human colonic microbiota: introducing the concept of prebiotics. J Nutr. 1995;125:1401-12*).*

The term "probiotic" means microbial cell preparations or components of microbial cells with a beneficial effect on the health or well-being of the host. (Salminen S, Ouwehand A. Benno Y. et al. "Probiotics: how should they be defined" Trends Food Sci. Technol. 1999:10 107-10). The microbial cells are generally bacteria or yeasts.

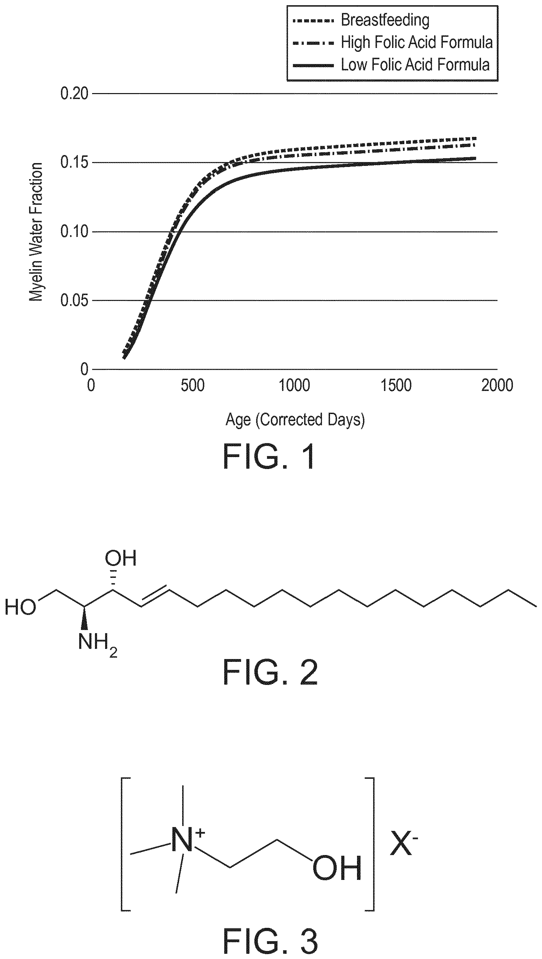

The term "sphingosine" indicates 2-amino-4-octadecene-1,3-diol having the structure described in Fig.2. Sphingosine may constitute the backbone of sphingolipids as below described.

The term "sphingoid bases" denotes sphingosine and a class of compounds structurally derived from it such as: dihydrosphingosine, dihydrosphingosine and phytosphingosine. The term "sphingoid bases" also includes the phosphorylated forms of dihydrosphingosine, sphingosine, dihydrosphingosine and phytosphingosine. In one embodiment, the sphingoid basis is selected from: dihydrosphingosine, sphingosine and phosphorylated forms thereof.

The term "sphingolipids" indicates a class of lipids containing a backbone of sphingoid bases wherein the amino group (-NH₂) is acylated with a fatty acid residue. "Ceramides" and "sphingomyelin" as below defined are exemplary classes of sphingolipids.

The term "ceramide" indicates a lipid molecule wherein the sphingosine backbone is acylated with a fatty acid residue. When the term ceramide is used in the present specifications, it may identify a single ceramide species as well as a mixture of single ceramide species. In particular ceramide is a compound of formula (IXa), or a mixture of compounds of formula (IXa) 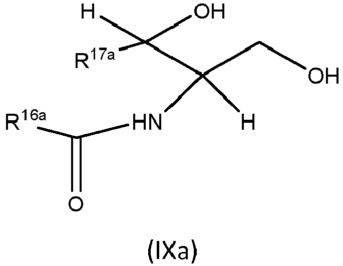 wherein,
R16a is a C2 to C43 branched or unbranched acyclic alkyl, or acyclic alkenyl group,
R17a is a C2 to C43 branched or unbranched acyclic alkyl, or acyclic alkenyl group.

More particularly, R16a is a C13 to C43 branched or unbranched acyclic alkyl, or acyclic alkenyl group which together with the adjacent carbonyl group corresponds to a C14 to C44 saturated or unsaturated fatty acid residue.

Non limiting examples of C14 to C44 saturated or unsaturated fatty acids from which the fatty acid residue may stem include; C14:0, C15:0, C16:0, C18:0, C20:0, C21:0, C22:0, C23:0, C24:1, C25:0, C28:1, C30:2, C30:1, C30:0, C32:3, C32:2, C32:1, C32:0, C33:1, C34:3, C34:2, C34:1, C34:0, C35:2, C35:0, C36:4, C36:3, C36:2, C36:1, C36:0, C37:1, C37:0, C38:4, C38:3, C38:1, C38:0, C39:1, C39:0, C40:2, C40:1, C40:0, C41:2, C41:1, C41:0, C42:47, C42:3, C42:2, C42:1, C42:0, C44:3, C44:1.

Even more particularly, R16a is a C13 to C23 branched or unbranched acyclic alkyl, or acyclic alkenyl group which together with the adjacent carbonyl group corresponds to a C14 to C24 saturated or unsaturated fatty acid residue, wherein the fatty acid from which the fatty acid residue stemmed is selected from the group consisting of; C14:0, C15:0, C16:0, C18:0, C20:0, C21:0, C22:0, C23:0, C24:0, C18:1n-9, C18:2n-6, and C24:1n-9, and more particularly the group consisting of C16:0, C18:0, C20:0, C22:0 and C24:0.

Even more particularly still, ceramide is a mixture of compounds of formula (IXa) wherein the mixture is such that the total number of fatty acid residues (R16a together with the adjacent carbonyl group) comprised in the mixture are predominately saturated fatty acids, and the least predominant are unsaturated fatty acids. More particularly the mixture will be such that that 80% to 96% of said fatty acid residues in the mixture are saturated fatty acids, in particular C14, C15, C16, C18, C20, C22, C23, C24 saturated fatty acids, more particularly C16, C18, C20, C22 and C24.

The term "ganglioside" as used herein indicates an oligoglycosylceramide lipid molecule comprising the residue of a ceramide of formula IXa as defined herein. When the term ganglioside is used in the present specifications, it may identify a single ganglioside species as well as a mixture of single ganglioside species comprising the residue of a ceramide of formula IXa as defined herein.

The term "sphingomyelin " indicates a lipid molecule wherein the sphingosine backbone is acylated with a fatty acid residue at the amino group (-NH₂) and wherein the hydroxyl group at position 1 of the sphingosine backbone is linked to a phospho-choline or phospho-ethanolamine group. When the term sphingomyelin is used in the present specifications, it may identify a single sphingomyelin species as well as a mixture of single sphingomyelin species wherein preferably the fatty acid residue is residue of a C14 to C44 fatty acid non limiting examples of which include Non limiting examples of C14 to C44 saturated or unsaturated fatty acids from which the fatty acid residue may stem include; C14:0, C15:0, C16:0, C18:0, C20:0, C21:0, C22:0, C23:0, C24:1, C25:0, C28:1, C30:2, C30:1, C30:0, C32:3, C32:2, C32:1, C32:0, C33:1, C34:3, C34:2, C34:1, C34:0, C35:2, C35:0, C36:4, C36:3, C36:2, C36:1, C36:0, C37:1, C37:0, C38:4, C38:3, C38:1, C38:0, C39:1, C39:0, C40:2, C40:1, C40:0, C41:2, C41:1, C41:0, C42:47, C42:3, C42:2, C42:1, C42:0, C44:3, C44:1. More Preferably the fatty acid residue is a residue of a C16, C18, C20, C22 or C24 saturated fatty acid.

The term "choline" identifies quaternary ammonium salts containing the *N,N,N-*trimethylethanolammonium cation and having the structure reported in Fig 3. Within the context of the present invention, the term "choline" should be intended to identify all the choline present in the nutritional compositions on the invention, either in free form (or as a salt thereof) or as deriving from structures comprising it such as for example: phosphatidylcholine, choline hydroxide or sphingomyelin. More preferably the term "choline" is intended to identify all the choline present in the nutritional compositions on the invention in free form or as a salt thereof e.g choline hydroxide.

The term "**fatty acid derivative"** as used herein refers to a compound comprising a fatty acid, other than a phospholipid, and in particular to a free fatty acid, and/or a monoacylglycerol (hereinafter MAG), and/or a diacylglycerol (hereinafter DAG), and/or a triacylgylcerol (hereinafter TAG) and/or a cholesterol ester. More particularly the term refers to a MAG, DAG, TAG and/or a cholesterol ester. Even more particularly the term refers to a TAG.

The term "**MAG**" as used herein refers to a glycerol molecule in which one of the OH groups has formed an ester bond with a fatty acid. In particular the term "MAG" as used herein refers to a compound of formula (X) 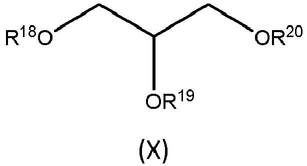 Wherein,
two of R¹⁸ R^{19 or} R²⁰ are H and wherein one of R¹⁸ R^{19 or} R²⁰ is a C4 to C44 saturated or unsaturated acyl group.

More particularly, two of R¹⁸ R^{19 or} R²⁰ are H and one of R¹⁸ R¹⁹ or R²⁰ is a C10 to C24 saturated or unsaturated acyl group, and even more particularly a C14 to C24 saturated or unsaturated acyl group.

The term "**DAG**" as used herein refers to glycerol molecule in which two of the OH groups have formed an ester bond with two fatty acids. In particular the term "DAG" as used herein refers to a compound of formula (X)
Wherein,
one of R¹⁸ R¹⁹ or R²⁰ are H and wherein two of R¹⁸ R^{19 or} R²⁰ are C4 to C44 saturated or unsaturated acyl groups. More particularly C10 to C24 saturated or unsaturated acyl groups, and even more particularly C14 to C24 saturated or unsaturated acyl groups. The two C4 to C44 saturated or unsaturated acyl groups may be the same or different.

The term "**TAG**" as used herein refers to glycerol molecule in which three of the OH groups have formed an ester bond with three fatty acids. In particular the term "TAG" as used herein refers to a compound of formula (X)
Wherein,
Wherein all R¹⁸ R^{19 or} R²⁰ are C4 to C44 saturated or unsaturated acyl groups, more particularly C10 to C24 saturated or unsaturated acyl groups, and even more particularly C14 to C24 saturated or unsaturated acyl groups. The three C4 to C44 saturated or unsaturated acyl groups may all be the same, all different, or two may be the same and one different.

The term "**cholesterol ester**" as used herein refers to a compound of formula (XI) 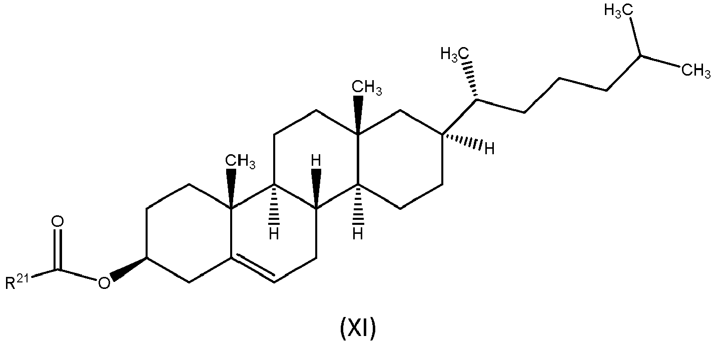 Wherein,
R²¹ is a C2 to C43 branched or unbranched acyclic alky, or acyclic alkenyl group.

More particularly, R²¹ is a C9 to C43 branched or unbranched acyclic alkyl, or acyclic alkenyl groups which together with the adjacent carbonyl group corresponds to a C10 to C44 saturated or unsaturated fatty acid residue, and even more particularly a C14 to C24 saturated or unsaturated fatty acid residue

The term "**fatty acid**" as used herein refers to a compound of formula (XII) 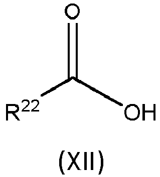 Wherein
R²² is a C2 to C43 branched or unbranched acyclic alkyl, or acyclic alkenyl group.

More particularly, R²² is a C9 to C43 branched or unbranched acyclic alkyl, or acyclic alkenyl group, and even more particularly a C13 to C23 branched or unbranched acyclic alkyl, or acyclic alkenyl group.

Within the context of the present invention the term "DHA" identifies docosahexaenoic acid. Within the context of the present invention, the term "DHA" should be intended to identify all the DHA present in the nutritional compositions on the invention, either in free form (as a fatty acid or a physiologically acceptable slat thereof) or comprised in a fatty acid derivative structure.

Within the context of the present invention, the term "ARA" or "AA" identifies arachidonic acid. Within the context of the present invention, the term "ARA" should be intended to identify all the ARA present in the nutritional compositions on the invention, either in free form (as a fatty acid or a physiologically acceptable slat thereof) or comprised in a fatty acid derivative structure.

The term "**vitamin**" as used herein refers to any vitamin. Non limiting examples of vitamins include: vitamin A, vitamin B1, vitamin B2, vitamin B6, vitamin K, vitamin C, vitamin D, niacin, biotin, pantothenic acid, folic acid, vitamin B12, and combinations thereof.

Within the context of the present invention, the term "folic acid" is to be intended as identifying all the folic acid present in the nutritional compositions of the invention either as such or in the form of one physiologically acceptable salt thereof (folate) and mixtures thereof.

Within the context of the present invention, the term **"mineral"** as used herein refers to any mineral. Non limiting examples of minerals include: iron, zinc, calcium, phosphorus, copper, magnesium iodine, manganese, chloride, potassium, sodium, selenium, chromium, and combinations thereof. Minerals are usually added in salt form.

Within the context of the present invention the term "Iron" is to be intended as identifying all the iron present in the nutritional compositions of the invention either in free form, or in the form of a physiologically acceptable (salt such as, for example: ferric citrate, ferric phosphate, ferric pyrophosphate, ferrous ascorbate, ferrous carbonate, ferrous citrate, ferrous fumarate, ferrous gluconate, ferrous lactate, ferrous sulfate or mixtures thereof) or in the form of one physiologically acceptable iron complex (such as for example EDTA ferric sodium salt) and mixtures thereof.

Within the context of the present invention, the term **"phospholipid"** as used herein refers to any phospholipid, and in particular a compound of formula (I) 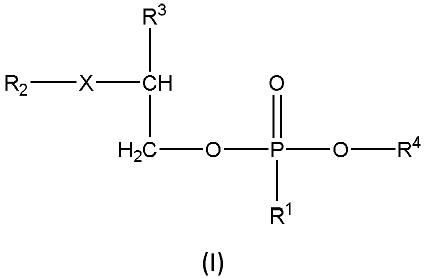 wherein,
R¹ is O;
X is NH or O;
R² is a C2-C44 saturated or unsaturated, linear or branched acyl group;
R³ is a substituent of formula (II) or formula (III): 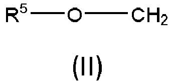 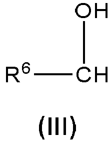
Wherein, R⁵ is a C2-C44 saturated or unsaturated, linear or branched acyl group and
R⁶ is a C2-C44 saturated alkyl or alkenyl group; and
R⁴ is selected from; a C5 or C6 substituted or unsubstituted cyclic alkyl or alkenyl group, or,
-(CH2)n-R⁷, wherein n is an integer ranging from 1 to 4, in particular 1 to 2 and R⁷ is -N(CH3)3+, NH3+, or a substituent of formula (IV) and, 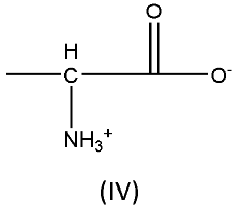
in particular R⁴ is a C6 cyclic alkyl or alkyl or alkenyl group substituted with one or more hydroxy groups, more particular R⁴ is derived from inositol (C6H12O6), and even more particularly myo-inositol i.e. R⁴ is:

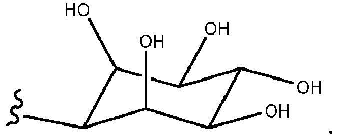

As used herein the term **"acyclic"** refers to a group that is not cyclic, i.e. does not contain a closed chain of atoms.

Within the context of the present invention, the term **"Phosphatidylinositole"** indicates a compound of formula (V) 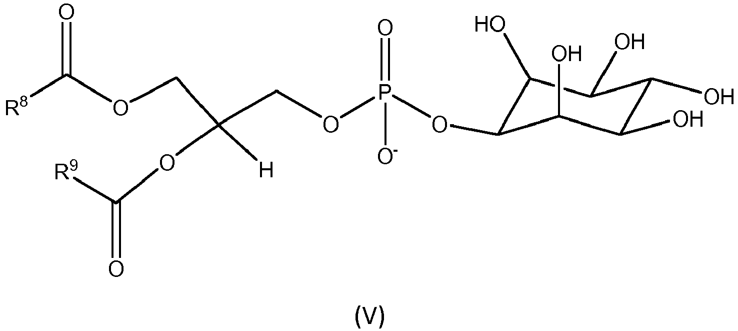
Wherein R⁸ is a C2 to C43 branched or unbranched acyclic alkyl, or acyclic alkenyl group and,
R⁹ is a C2 to C43 branched or unbranched acyclic alkyl, or acyclic alkenyl group.

More particularly R⁸ and R⁹ are, independently of each other, C13 to C43 branched or unbranched acyclic alkyl, or acyclic alkenyl groups which together with their adjacent carbonyl group are correspond to C14 to C44 saturated or unsaturated fatty acid residues, and even more particularly R⁸ and R⁹ are, independently of each other, C13 to C23 branched or unbranched acyclic alkyl, or acyclic alkenyl groups which together with their adjacent carbonyl group correspond to C14 to C24 saturated or unsaturated fatty acid residues.

More particularly, R⁸ and R⁹ are C13 to C23 branched or unbranched acyclic alkyl, or acyclic alkenyl groups which together with their adjacent carbonyl group are C14 to C24 saturated or unsaturated fatty acid residues, wherein the fatty acids from which the fatty acid residues stem are selected from the group consisting of; C14:0, C15:0, C16:0, C18:0, C20:0, C20:3, C20:4, C21:0, C22:0, C23:0, C24:0, C18:1n-9, C18:2n-6, and C24:1n-9. Even more particularly C18:0, C18:1n-9, C18:2, C20:3, and C20:4.

Within the context of the present invention, the term **"Phosphatidylserine"** indicates a compound of formula (VI) 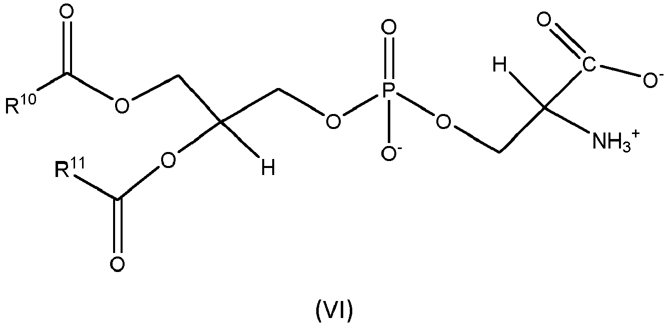
Wherein R¹⁰ is a C2 to C43 branched or unbranched acyclic alkyl, or acyclic alkenyl group and,
R¹¹ is a C2 to C43 branched or unbranched acyclic alkyl, or acyclic alkenyl group.

More particularly, R¹⁰ and R¹¹ are, independently of each other, C13 to C43 branched or unbranched acyclic alkyl, or acyclic alkenyl groups which together with their adjacent carbonyl group correspond to C14 to C44 saturated or unsaturated fatty acid residues, and even more particularly R¹⁰ and R¹¹ are, independently of each other, C13 to C23 branched or unbranched acyclic alkyl, or acyclic alkenyl groups which together with their adjacent carbonyl group correspond to C14 to C24 saturated or unsaturated fatty acid residues.

More particularly, R¹⁰ and R¹¹ are C13 to C23 branched or unbranched acyclic alkyl, or acyclic alkenyl groups which together with their adjacent carbonyl group are C14 to C24 saturated or unsaturated fatty acid residues, wherein the fatty acids from which the fatty acid residues stem are selected from the group consisting of; C14:0, C15:0, C16:0, C18:0, C20:0, C20:3, C20:4, C21:0, C22:0, C23:0, C24:0, C18:1n-9, C18:2n-6, and C24:1n-9. Even more particularly C18:0, C18:1n-9, C20:4, and C22:6.

Within the context of the present invention, the term **"Phosphatidylethanolamine"** indicates a compound of formula (VII) 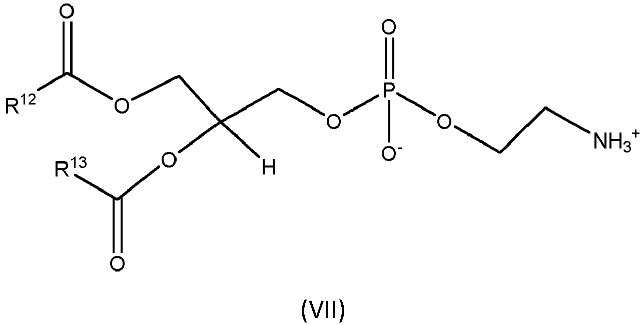
Wherein R¹² is a C2 to C43 branched or unbranched acyclic alkyl, or acyclic alkenyl group and,
R¹³ is a C2 to C43 branched or unbranched acyclic alkyl, or acyclic alkenyl group.

More particularly, R¹² and R¹³ are, independently of each other, C13 to C43 branched or unbranched acyclic alkyl, or acyclic alkenyl groups which together with their adjacent carbonyl group correspond to C14 to C44 saturated or unsaturated fatty acid residues, and even more particularly R¹² and R¹³ are, independently of each other, C13 to C23 branched or unbranched acyclic alkyl, or acyclic alkenyl groups which together with their adjacent carbonyl group correspond to C14 to C24 saturated or unsaturated fatty acid residues.

Within the context of the present invention, the term **"Phosphatidylcholine"** identifies a compound of formula (IX) 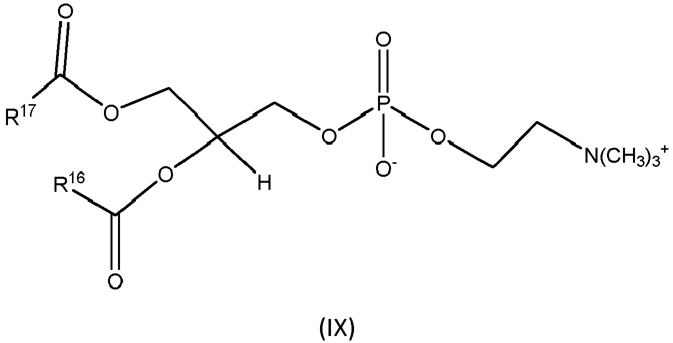
Wherein R¹⁶ is a C2 to C43 branched or unbranched acyclic alkyl, or acyclic alkenyl group and,
R¹⁷ is a C2 to C43 branched or unbranched acyclic alkyl, or acyclic alkenyl group.

More particularly, R¹⁶ and R¹⁷ are, independently of each other, C13 to C43 branched or unbranched acyclic alky, or acyclic alkenyl groups which together with their adjacent carbonyl group correspond to C14 to C44 saturated or unsaturated fatty acid residues, and even more particularly R¹⁶ and R¹⁷ are, independently of each other, C13 to C23 branched or unbranched acyclic alkyl, or acyclic alkenyl groups which together with their adjacent carbonyl group correspond to C14 to C24 saturated or unsaturated fatty acid residues.

More particularly, R¹⁶ and R¹⁷ are C13 to C23 branched or unbranched acyclic alkyl, or acyclic alkenyl groups which together with their adjacent carbonyl group are C14 to C24 saturated or unsaturated fatty acid residues, wherein the fatty acids from which the fatty acid residues stem are selected from the group consisting of; C14:0, C15:0, C16:0, C16:1, C18:0, C20:0, C20:1, C20:3, C20:4, C21:0, C22:0, C22:6, C23:0, C24:0, C18:1n-9, C18:2n-6, and C24:1n-9. Even more particularly C14:0, C16:0, C18:0, C18:1n-9, C18:2n-6, C20:1, C20:3, C20:4, and C22:6.

The term "cfu" should be understood as colony-forming unit.

Exclusive breast feeding / infants or young children exclusively breast fed: has the common meaning of infants for which great majority of nutrients and/or energy originates from human breast milk (the "great majority" can be at least 90% or at least 95%, or at least 99%).

Infants/young children predominantly fed infant formula: has the common meaning and refers to infants or young children which nutritional sources of nutrients and/or energy predominantly originates from synthetic infant formula, follow-on milk or growing-up milks. Predominantly refers to at least 50% of those nutrients and/or energy, or at least 75%.

All percentages are by weight unless otherwise stated.

The invention will now be described in further details. It is noted that the various aspects, features, examples and embodiments described in the present application may be compatible and/or combined together.

In addition, in the context of the invention, the terms "comprising" or "comprises" do not exclude other possible elements. The composition of the present invention, including the many embodiments described herein, can comprise, consist of, or consist essentially of the essential elements and limitations of the invention described herein, as well as any additional or optional ingredients, components, or limitations described herein or otherwise depending on the needs.

The terms "in particular" or "more particularly" as used herein should not be considered limiting but should be interpreted as being synonymous with "for example" or "especially".

### Detailed description of the invention

For the sake of clarity, all the embodiments and aspects reported hereafter will be applicable to different embodiments and aspects described for the present invention *mutatis mutandis.*

Unless otherwise indicated, all amounts indicated for nutrients are expressed as amounts per weight of dry nutritional composition.

Where a nutrient may be comprised in a composition under different forms (as such or in the form of salts, complexes or more complex structures comprising the nutrient) the amounts reported hereafter are to be intended to make reference to the amount of the nutrient as such.

The nutritional composition according to the present invention comprise a vitamin or mixtures thereof.

Particularly effective vitamins may be folic acid, vitamin B12 and vitamin B6, in particular folic acid and vitamin B12, in particular folic acid.

A vitamin may be comprised in a composition of the invention in an amount from 0.001% up to 99.999% of the composition.

In an embodiment the composition of the invention comprises vitamin B12 and/or folic acid.

Folic acid may be comprised in the composition of the invention in an amount constituting 0.001% up to 99.999% of the composition.

In particular folic acid may be comprised in an amount of higher than 50mcg/100g of the dry composition, more particularly 50mcg to 500mcg/100g of the dry composition.

The nutritional composition according to the present invention comprise folic acid in addition to a to a mineral or mixtures thereof, for example iron, zinc, calcium, phosphorus, and/or magnesium.

Folic acid may be comprised in the composition of the invention in an amount constituting 0.001% up to 99.999% of the composition.

The nutritional composition according to the present invention comprises folic acid. In one embodiment, the nutritional composition according to the present invention comprise folic acid in an amount higher than 50 mcg/100g .

In one embodiment, the nutritional composition according to the present invention comprise folic acid in an amount higher than 110 mcg/100g.

In one embodiment, the nutritional composition according to the present invention comprise folic acid in an amount ranging from 50 to 500 mcg/100g or from 50 to 400 mcg/100g.

In a further embodiment, the nutritional composition according to the present invention comprise folic acid in an amount ranging from 110 to 500 mcg/100g or from 110 to 400 mcg/100g.

In a still further embodiment, the nutritional composition according to the present invention comprise folic acid in an amount ranging from 110 to 400 mcg/100g or from 110 to 350 mcg/100g.

In one embodiment, the nutritional composition according to the present invention comprises levels of folic acid such that the total daily intake derived from the nutritional composition of the invention will not exceed 400mcg.

Folic acid may be incorporated in the nutritional compositions of the invention as such or in the form of one physiologically acceptable salt thereof (folate) or mixtures thereof.

In another embodiment, the nutritional composition according to the present invention comprise also Vitamin B12.

Vitamin B12 may be comprised in the composition of the invention in an amount constituting from 0.001% up to 99.999% of the composition.

In particular vitamin B12 may be comprised in the composition in an amount of selected from the group consisting of; higher than 0.01mcg, in particular higher than 0.04mcg, in particular higher than 0.05mcg, wherein all weights are/100g of the dry composition.

In an embodiment the composition of the invention comprises Vitamin B12 in an amount selected from the group consisting of; higher than 0.5 mcg, ranging from 0.1 to 10 mcg, 0.4 to 5mcg, 0.5 to 2mcg, 1 to 1.5mcg, 4 to 8.5mcg, wherein all weights are per 100g of the dry composition.

In an embodiment, the composition according to the present invention comprises an amount of vitamin B12 such that the total daily intake derived from the nutritional composition of the invention will not exceed 7.6 mcg/100g of the dry composition (77.6 mcg/Kg of the dry composition).

Vitamin B12 may be incorporated in the nutritional compositions of the invention as such or in the form of a physiologically acceptable salt thereof or mixtures thereof, or via any source comprising vitamin B12. In particular vitamin B12 may be incorporated into the composition in its pure form, as cyanocobalamin, hydroxocobalamin, and any combination thereof.

In one embodiment, the nutritional composition according to the present invention comprises one or more of the following ingredients in addition to a mineral mixtures thereof, for example in addition vitamins: minerals and/or phospolipids and/or fatty acids and/or choline.

It may be particularly beneficial if the composition of the invention further comprises one or more of the following ingredient: minerals and/or phospholipids and/or fatty acid derivatives and/or choline.

In one embodiment, the nutritional composition according to the present invention comprise a phospholipid, a metabolic precursor and/or a metabolite thereof (for example sphingomyelin, phosphatidylcholine and/or phosphatidylinositol) in addition to a vitamin or mixtures thereof.

A phospholipid, a metabolic precursor and/or metabolite thereof may be comprised in a composition in an amount up to 99.999% of the composition.

In particular sphingomyelin, a metabolic precursor and/or metabolite thereof may be comprised in a composition in an amount up to 99.999% of the composition.

More particularly the composition will comprise sphingomyelin in an amount higher than 200mg/kg of the dry weight of the composition, more particularly ranging from 200mg to 2.5g/kg of the dry weight of the composition.

In an embodiment the composition comprises sphingomyelin in an amount selected from the group consisting of; higher than 200 mg/kg, higher than 300 mg/kg, ranging from 200 mg to 2.5 g/kg, ranging from 200mg to 2g/kg, in amount ranging from 300 mg to 1.5 g /kg or from 400 mg to 1g/Kg. All weights being per dry weight of the composition.

In one embodiment, phosphatidylcholine, a metabolic precursor and/or metabolite thereof may be comprised in a composition in an amount up to 99.999% of the composition.

More particularly the composition will comprise phosphatidylcholine in an amount higher than 200mg/kg of the dry weight of the composition, more particularly ranging from 200mg to 2.5g/kg of the dry weight of the composition.

In an embodiment the composition comprises phosphatidylcholine in an amount selected from the group consisting of; higher than 200 mg/kg, higher than 300 mg/kg, ranging from 200 mg to 2.5 g/kg, ranging from 200mg to 2g/kg, in amount ranging from 300 mg to 1.5 g /kg or from 400 mg to 1g/Kg. All weights being per dry weight of the composition.

In particular phosphatidylinositole, a metabolic precursor and/or metabolite thereof may be comprised in a composition in an amount up to 99.999% of the composition.

More particularly the composition will comprise phosphatidylinositole in an amount higher than 200mg/kg of the dry weight of the composition, more particularly ranging from 200mg to 1.5g/kg of the dry weight of the composition.

In an embodiment the composition comprises phosphatidylinositole in an amount selected from the group consisting of; higher than 200 mg/kg, higher than 300 mg/kg, ranging from 200 mg to 2.5 g/kg, ranging from 200mg to 2g/kg, in amount ranging from 250mg to 800mg /kg or from 400 mg to 1.5g/Kg. All weights being per dry weight of the composition.

In particular phosphatidylserine, a metabolic precursor and/or metabolite thereof may be comprised in a composition in an amount up to 99.999% of the composition.

More particularly the composition will comprise phosphatidylserine in an amount higher than 150mg/kg of the dry weight of the composition, more particularly ranging from 200mg to 1.5g/kg of the dry weight of the composition.

In an embodiment the composition comprises phosphatidylserine in an amount selected from the group consisting of; higher than 150, higher than 200 mg/kg, higher than 300 mg/kg, ranging from 200 mg to 2.5 g/kg, ranging from 200mg to 2g/kg, in amount ranging from 250mg to 1000mg /kg or from 400 mg to 1g/Kg. All weights being per dry weight of the composition.

In particular phosphatidylethanolamine, a metabolic precursor and/or metabolite thereof may be comprised in a composition in an amount up to 99.999% of the composition.

More particularly the composition will comprise phosphatidylethanolamine in an amount higher than 150mg/kg of the dry weight of the composition, more particularly ranging from 150mg to 1.5g/kg of the dry weight of the composition.

In an embodiment the composition comprises phosphatidylethanolamine in an amount selected from the group consisting of; higher than 170mg/kg, higher than 180 mg/kg, higher than 200 mg/kg, ranging from 200 mg to 2.5 g/kg, ranging from 200mg to 2g/kg, in amount ranging from 250mg to 800mg /kg or from 200 mg to 1g/Kg. All weights being per dry weight of the composition.

In an embodiment the composition of the invention comprises phospholipids including phosphatidylinositole, phosphatidylserine, phosphatidylethanolamine, sphingomyelin and phosphatidylcholine such that the total concentration does not exceed 15.4g/kg.

If a metabolic precursor and/or metabolite of one or more phospholipid is used in a composition in place of or in combination with a phospholipid, said compounds may be used in amounts such that the level of phospholipids physiologically delivered by said composition is in line with those set out hereinabove. It is well within the purview of the skilled person to determine appropriate amounts.

The term metabolic precursor and/or metabolite of one or more phospholipid as used herein does not include choline.

Non limiting examples of metabolic precursors and/or a metabolite of phospholipids, in particular sphingomyelin, phosphatidylcholine, phosphatidylinositole, phosphatidylserine and/or phosphatidylethanolamine are: galactoceramides, glucoceramides, sphingosine, sphingosine-1-phosphate, ceramide, D-erythro-dihydroceramide and ceramide-1-phosphate, and gangliosides.

Particularly effective phospholipids may be phosphatidylcholine, phosphatidylserine, phosphatidylinositol and/or sphingomyelin, in particular sphingomyelin.

In an embodiment of the present invention the phospholipid is phosphatidylcholine, phosphatidylserine, phosphatidylinositol, sphingomyelin and/or a metabolic precursor and/or metabolite of any of the foregoing and/or combinations of any of the foregoing. In particular the phospholipid is sphingomyelin, a metabolic precursor and/or metabolite thereof.

Particularly effective metabolic precursors and/or a metabolite of phospholipids, in particular sphingomyelin include ceramide and gangliosides and ceramide -1-phosphate and d-erythro-dihydroceramide.

Particularly effective gangliosides may be monosialoganglioside-3 (GM3) gangliosides and/or disialogangliosides 3 (GD3) gangliosides.

Ceramide -1-phosphate and d-erythro-dihydroceramide will comprise a residue of a ceramide of formula IXa as defined herein.

Gangliosides and/or ceramides and/or Ceramide -1-phosphate and/or d-erythro-dihydroceramide may be comprised in the composition in any amount.

Concentrations in the range of 2-11.5mg/100g of GD3 and/or GM3 may be particularly effective.

Spingomyelin may be synthesised from ceramide and/or one or more ganglioside and phosphatidylcholine, accordingly, it may be particularly beneficial if ceramide and/or one or more ganglioside is used in combination with phosphatidylcholine a metabolic precursor or metabolite thereof.

The phospholipid, metabolic precursors and/or metabolite thereof, comprised in the composition of the invention may be natural, synthetic or a mixture thereof. Said metabolic precursors and/or a metabolite, may be used in the composition of the invention in their pure form, or substantially pure form. Alternatively, they may be added in the form of a source comprising them.

Any source of a phospholipid metabolic precursors and/or metabolite thereof, suitable for ingestion by a subject for which the composition is intended to be consumed may be used in the invention.

In particular the phospholipid a metabolic precursor or metabolite thereof, will come from natural sources, non limiting examples of which include, eggs, soy, bovine brains, and/or mammalian milk or extracts thereof. Non limiting examples of soy sources include soy lecithin-food additive, non limiting examples of mammalian milk include bovine, camel, sheep, goat milk including skilled milks. Non limiting extracts of milk include protein extracts e.g. whey protein and casein, milk fat globule membranes (MFGM) and extracts comprising them.

A particularly useful source of a phospholipids a metabolic precursor or metabolite thereof, in particular sphingomyelin, that may be used in the present invention may be a bovine milk whey protein concentrate enriched in alpha-lactalbumin, and/or none pure alpha-lactalbumin which has been extracted from milk whey protein, in particular bovine milk whey protein.

Alpha-Lactalbumin is a high-quality, easy-to-digest whey protein and is the primary protein found in HM. Alpha-lactalbumin and/or an alpha-lactalbumin enriched milk fraction is ideal for use in lower protein infant formulas due to its high content of essential amino acids, particularly tryptophan. Although alpha-Lactalbumin is in itself a protein non pure sources may comprise sphingomyelin.

In an embodiment a phospholipid a metabolic precursor or metabolite thereof, in particular sphingomyelin, is used in the form of a whey protein concentrate enriched in alpha-lactalbumin or as alpha-lactalbumin.

In a more particular embodiment, a whey protein concentrate enriched in alpha-lactalbumin or alpha-lactalbumin having a phospholipid content, in particular sphingomyelin content higher than 500 mg/100g dry weight of the composition is used.

Another particularly useful source of phospholipids a metabolic precursor, or metabolite thereof, may be MFGM or extracts comprising them, in particular MFGM, or extracts comprising them from bovine milk. It may be particularly beneficial if the MFGM or extracts comprising them comprises at least 1%, 2%, 5%, 10%, 20%, 30%, 40% phospholipids and/or at least 0.1%, 0.2%, 0.5% to 5%, 0.8% to 3%, 1% to 2%, 1.6%, 1.9%, 1.8% of phosphatidylcholine, phosphatidylinositole, phosphatidylserine, phosphatidylethanolamine, and/or sphingomyelin. The MFGM may also further comprise magnesium, phosphorus and or calcium, in particularly in concentrations ranging from 0.05% to 2%, 0.1% to 0.4%.

The person skilled in the art may identify appropriate amounts of the above mentioned nutrients, metabolic precursors or metabolites thereof based on the nature, purpose, the target subject and the dosage of the composition e.g. how many times per day the composition is to be ingested by the subject. Typically an effective dose will depend on age, size and health status of the subject, on the subject's lifestyle, the amounts of nutrients in the composition, and maybe on the gender of the subject.

In one embodiment, the nutritional composition according to the present invention comprises sphingomyelin in addition to a vitamin (for example folic acid or Vitamin B12) or mixtures thereof.

In one embodiment, the nutritional composition according to the present invention comprises sphingomyelin in an amount higher than 200 mg/kg.

In another embodiment, the nutritional composition of the invention comprises sphingomyelin in an amount higher than 300 mg/kg.

In still further embodiment, the nutritional composition according to the present invention comprises sphingomyelin in an amount ranging from 200 mg to 2,5 g/kg.

In another embodiment, the nutritional composition according to the present invention comprises sphingomyelin in an amount ranging from 200 to 2g/kg, for example from 300 mg to 1,5 g /kg or from 400 mg to 1g/Kg.

Within the context of the present invention, Sphingomyelin may be administered as such or in the form of a metabolic precursors and/or a metabolite thereof. Without wishing to be bound by theory, it is considered that such metabolic precursors and/or metabolites elicit the same beneficial effect in promoting and/or supporting an optimal myelination trajectory in the brain or preventing a suboptimal myelination trajectory in the brain as the direct administration of sphingomyelin does. Non limiting exemplary species in this respect are: galactoceramides, glucoceramides, sphingosine, sphingosine-1-phosphate, ceramide, D-erythro-dihydroceramide and ceramide-1-phosphate and gangliosides.

The nutritional compositions of the invention comprise sphingomyelin and/or metabolic precursors or metabolites thereof.

Sphingomyelin is present in natural sources such as: alpha-lactalbumin, egg, bovine brain, or bovine milk etc.

A sphingomyelin and/or metabolic precursors or metabolites thereof may be incorporated in the nutritional compositions of the invention as a single species, as an ingredient consisting of a mixture of different sphingomyelin species or by addition of a natural or synthetic ingredient comprising one ore more sphingomyelin species.

In one embodiment, sphingomyelin may be incorporated in the nutritional compositions of the invention as comprised in skim milk powder, alpha lactalbumin, whey protein concentrate and/or whey protein concentrate enriched in alpha-lactalbumin. In one embodiment, the whey protein concentrate enriched in alpha-lactalbumin may have a sphingomyelin content higher than 500 mg/100g.

In one embodiment, sphingomyelin may be incorporated in the nutritional compositions of the invention as a single ingredient.

In one embodiment, the nutritional composition according to the present invention comprises metabolic precursors and/or metabolites of sphingomyelin in amounts such that they would deliver physiologically the same sphingomyelin level as those delivered by a nutritional composition comprising from 200 mg to 2,5 g/kg of sphingomyelin. In another embodiment, the nutritional composition according to the present invention comprises metabolic precursors and/or metabolites of sphingomyelin in amounts such that they would deliver physiologically the same sphingomyelin level as those delivered by a nutritional composition comprising from 400 mg to 1,5g/Kg of sphingomyelin.

Such amounts would be derived by the skilled person on the basis of his knowledge in the field.

In one embodiment, the nutritional composition according to the present invention comprise a mineral or mixtures thereof.

Particularly effective minerals may be iron, zinc, calcium, phosphorus, copper, and magnesium, in particular iron.

In an embodiment the composition of the invention comprises iron and/or zinc and/or calcium and/or phosphorus and/or copper and/or and magnesium, in particular iron and zinc, more particularly iron.

In another embodiment, the nutritional composition according to the present invention comprise also Iron in addition to folic acid.

In one embodiment, the nutritional composition according to the present invention comprise Iron in an amount higher than 5 mg/100g.

In one embodiment, the nutritional composition according to the present invention comprise Iron in an amount higher than 9 mg/100g.

In one embodiment, the nutritional composition according to the present invention comprise Iron in an amount ranging from 5 to 40 mg/100g or from 9 to 40 mg/100g. In another embodiment, the nutritional composition according to the present invention comprise Iron in an amount ranging from 5 to 20 mg/100g or from 9 to 20 mg/100g.

In another embodiment, the nutritional composition according to the present invention comprise Iron in an amount ranging from 5 to 15 mg/100g or from 9 to 15 mg/100g.

The nutritional compositions of the invention may comprise Iron in addition to folic acid.

Iron may be incorporated in the nutritional compositions of the invention in the form of one physiologically acceptable salt such as, for example: ferric citrate, ferric phosphate, ferric pyrophosphate, ferrous ascorbate, ferrous carbonate, ferrous citrate, ferrous fumarate, ferrous gluconate, ferrous lactate, ferrous sulfate or mixtures thereof.

Iron may be incorporated in the nutritional compositions of the invention in the form of one physiologically acceptable iron complex (such as for example EDTA ferric sodium salt) or mixtures thereof.

In one embodiment, the nutritional composition according to the present invention comprises levels of iron such that the total daily intake derived from the nutritional composition of the invention will not exceed 40 mg.

In another embodiment, the nutritional composition according to the present invention comprise Zinc.

Zinc may be comprised in the composition of the invention in an amount constituting from 0.001% up to 99.999% of the composition.

In particular zinc may be comprised in the composition in an amount higher than 0.08mg, higher than 0.3mg, higher than 0.5mg, wherein all weights are/100g of the dry composition.

In an embodiment, the composition according to the present invention comprises zinc in an amount selected ranging from 0.5 to 8mg, 2 to 5.5mg, 2.5 to 4.5mg, 3 to 4mg, 4 to 7.5mg, wherein all weights are per 100g of the dry composition.

In an embodiment, the composition according to the present invention comprises levels of zinc such that the total daily intake derived from the nutritional composition of the invention will not exceed 302.4mg/day, or will not exceed 245mg/day, or will not exceed 166mg/day, or will not exceed 98.9mg/day, or will not exceed 95.6mg/day.

Zinc may be incorporated in the compositions of the invention in the form of a physiologically acceptable salt such as, for example: zinc nitrate, zinc sulfate, zinc gluconate, zinc acetate or mixtures thereof, or in the form of a physiologically acceptable zinc complex (such as for example zinc picolinate) or mixtures thereof.

In another embodiment, the nutritional composition according to the present invention comprise Calcium.

Calcium may be comprised in the composition of the invention in an amount from 0.001% up to 99.999% of the composition.

In particular calcium may be comprised in the composition in an amount higher than 0.84mg. higher than 2.52mg, higher than 4.62mg, wherein all weights are /100g dry weight of the composition.

In an embodiment, the composition according to the present invention comprises calcium in an amount ranging from 84 to 760mg, ranging from 200 to 550mg, ranging from 250 to 450mg, ranging from 280 to 520, 350 to 650mg, wherein all weights are per 100g of the dry composition.

In an embodiment, the composition according to the present invention comprises levels of calcium such that the total daily intake derived from the nutritional composition of the invention will not exceed 482mg/day, or will not exceed 477mg/day.

Calcium may be incorporated in the composition of the invention as such or in the form of a physiologically acceptable salt and/or via any source comprising calcium. For example calcium carbonate, calcium chloride, calcium salts of citric acid, calcium gluconate, calcium glycerophosphate, calcium lactate, calcium hydroxide, calcium salts of orthophosphoric acid.

In another embodiment, the nutritional composition according to the present invention comprise Magnesium.

Magnesium may be comprised in the composition of the invention in an amount from 0.001% up to 99.999% of the composition.

In particular magnesium may be comprised in the composition in an amount higher than 0.2mg, higher than 0.35mg, higher than 0.5mg, wherein all weights are /100g dry weight of the composition.

In an embodiment, the composition according to the present invention comprises magnesium in an amount selected from the group consisting of; ranging from 0.35 to 90mg, ranging from 25 to 70mg, 30 to 65mg, 35 to 60mg, 40 to 50mg, 35 to 55mg, wherein all weights are per 100g of the dry composition.

In an embodiment, the composition according to the present invention comprises levels of magnesium such that the total daily intake derived from the nutritional composition of the invention will not exceed 110mg/day, or will not exceed 65mg/day.

Magnesium may be incorporated in the composition of the invention as such or in the form of a physiologically acceptable salt and/or via any source comprising magnesium. For example magnesium carbonate, magnesium chloride, magnesium oxide, magnesium sulphate, magnesium gluconate, magnesium hydroxide, magnesium salts of citric acid, magnesium salts of orthophosphoric acid.

In another embodiment, the nutritional composition according to the present invention comprise Phosphorus.

Phosphorus may be comprised in the composition of the invention in an amount from 0.001% up to 99.999% of the composition.

In particular phosphorus may be comprised in the composition in an amount higher than 1.7mg, higher than 14.3mg, higher than 27.3mg /100g dry weight of the composition.

In an embodiment, the composition according to the present invention comprises phosphorus in an amount selected from the group consisting of; ranging from 17 to 516mg, ranging from 129 to 400mg, ranging from 140 to 390mg, ranging from 150 to 370mg, 160 to 365mg, ranging from 270 to 350mg, 200 to 360mg, wherein all weights are per 100g of the dry composition.

In an embodiment, the composition according to the present invention comprises levels of phosphorus such that the total daily intake derived from the nutritional composition of the invention will not exceed 863mg/day, or will not exceed 787mg/day.

Phosphorus may be incorporated in the composition of the invention as such or in the form of a physiologically acceptable salt and/or via any source comprising phosphorus for example: calcium phosphate, calcium hydrogen phosphate

In another embodiment, the nutritional composition according to the present invention comprise Copper.

Copper may be comprised in the composition of the invention in an amount from 0.001% up to 99.999% of the composition.

In particular copper may be comprised in the composition in an amount higher than 10mcg, higher than 40mcg, higher than 60mcg, wherein all weight are/100g dry weight of the composition.

In an embodiment, the composition according to the present invention comprises copper in an amount selected from the group consisting of; higher than 100mcg, ranging from 100 to 850mcg, 180 to 650mcg, 200 to 400mcg, 210 to 300mcg, 210 to 240mcg, 450 to 850mcg, wherein all weights are per 100g of the dry composition.

In an embodiment, the composition according to the present invention comprises levels of copper such that the total daily intake derived from the nutritional composition of the invention will not exceed 1426mcg/day, or will not exceed 488mcg/day.

Copper may be incorporated in the composition of the invention as such or in the form of a physiologically acceptable salt and/or via any source comprising copper. For example copper may be incorporated into the composition as: copper sulfate and/or copper gluconate and/or copper carbonate, and/or copper citrate, and/or copper-lysine complex.

The nutritional compositions of the invention may comprise choline and/or metabolic precursors or metabolites thereof in addition to a vitamin (for example folic acid and/or Vitamin B12) or mixtures thereof.

In another embodiment, the nutritional composition according to the present invention comprise also choline in addition to folic acid.

In one embodiment, the nutritional composition according to the present invention comprise choline in an amount higher than 30 mg/100g.

In one embodiment, the nutritional composition according to the present invention comprise choline in an amount higher than 50 mg/100g or higher than 100 mg/100g . In one embodiment, the nutritional composition according to the present invention comprise choline in an amount ranging from 30 to 1000 mg/100g or from 30 to 700mg/100g.

In another embodiment, the nutritional composition according to the present invention comprise choline in an amount ranging from 50 to 1000 mg/100g or from 50 to 700mg/100g.

In a further embodiment, the nutritional composition according to the present invention comprise choline in an amount ranging from 50 to 500 mg/100g or from 100 to 400mg/100g.

In one embodiment, the nutritional composition according to the present invention comprises levels of choline such that the total daily intake derived from the nutritional composition of the invention will not exceed 1 g.

Choline may be incorporated in the nutritional compositions of the invention as such or in the form of one physiologically acceptable salt such as, for example: choline chloride, choline citrate, choline bitartrate or mixtures thereof.Within the context of the present invention, choline may be administered in combination with sphingomyelin as such or in the form of a metabolic precursors and/or a metabolite thereof such as for example phosphatidylcholine.

Choline and/or metabolic precursors or metabolites thereof may be incorporated in the nutritional compositions of the invention as single species, as an ingredient consisting of a mixture of different choline species or by addition of a natural or synthetic ingredient comprising one or more choline species.

The nutritional compositions of the invention may comprise choline and/or metabolic precursors or metabolites thereof in addition to folic acid.

In one embodiment, the nutritional composition according to the present invention also comprises a fatty acid derivative or mixtures thereof.

Non limiting examples of C10 to C44 saturated or unsaturated fatty acids that may be comprised in the fatty acid derivative i.e. that may be the free fatty acid or fatty acid from which the fatty acid residue(s) of the MAG, DAG, TAG and/or cholesterol ester may stem include; C10:0, C12:0, C14:0, C15:0, C16:0, C16:1n-7, C18:0, C18:1n-7, C18:1n-9, C18:2n-6, 18:3n-3, C20:0, C20:1n-9, C20:2n-6, C20:3n-6, C20:4n-6, 20:5n-3, C21:0, C22:0, C22:1n-9, C22:6n-3 C23:0, C24:1, in particular 24:1n-9, C25:0, C28:1, C30:2, C30:1, C30:0, C32:3, C32:2, C32:1, C32:0, C33:1, C34:3, C34:2, C34:1, C34:0, C35:2, C35:0, C36:4, C36:3, C36:2, C36:1, C36:0, C37:1, C37:0, C38:4, C38:3, C38:1, C38:0, C39:1, C39:0, C40:2, C40:1, C40:0, C41:2, C41:1, C41:0, C42:47, C42:3, C42:2, C42:1, C42:0, C44:3, C44:1. In particular said fatty acids will be selected from the group consisting of: C10:0, C12:0, C14:0, C16:0, C16:1n-7, C18:0, C18:1n-7, C18:1n-9, C18:2n-6, 18:3n-3, C20:0, C20:1n-9, C20:2n-6, C20:3n-6, C20:4n-6, 20:5n-3, C22:0, C22:1n-9, C22:6n-3, C24:1, 24:1n-9 in particular 24:1n-9.

It may be particularly beneficial if the fatty acid derivative comprises a saturated or unsaturated fatty acid selected from the group consisting of: C20:4n-6, C22:6n-3, C24:1n-9, C16:0, C18:1n-9, andC18.0. In particular C20:4n-6 and/or C22:6n-3 and/or C18:0. More particularly 22:6n-3 and/or C18:0.

Any fatty acid derivative suitable for ingestion by a subject for which the composition is intended to be consumed may be used in the invention.

In particular the fatty acid derivative will come from natural sources, non limiting examples of which include, eggs, algae, fish oil, mould, yeast, seeds, plants e.g. soy, and animal sources e.g. bovine brains, and/or mammalian milk or extracts thereof. Non limiting examples of soy sources include soy lecithin-food additive, non limiting examples of mammalian milk include bovine, camel, sheep, goat milk including skilled milks. Non limiting extracts of milk include protein extracts, milk fat globule membranes (MFGM) and extracts comprising them. Fatty acid derivatives may also come from palm oil, tallow, lard, cotton seed oil, peanut oil.

It may be particularly beneficial if the fatty acid derivative comprises a saturated or unsaturated fatty acid selected from the group consisting of: C20:4n-6, C22:6n-3, C24:1n-9, C16:0, C18:1n-9, and C18.0. In particular C20:4n-6 and/or C22:6n-3 and/or C18:0. More particularly 22:6n-3 and/or C18:0.

C20:4n-6 is arachidonic acid (herein after ARA or AA). C22:6n-3 is docosahexaenoic acid (hereinafter DHA). 24:1n-9 is nervonic acid. C18.0 is stearic acid. C16:0 is palmitic acid. C18:1n-9 is Oleic acid.

In an embodiment the composition according to the invention comprises fatty acid derivative comprising DHA and/or ARA and/or nervonic acid and/or stearic acid, in particularly a fatty acid derivative comprising DHA and/or ARA and/or Stearic acid. Most particularly a fatty acid derivative comprising DHA and/ or Stearic acid.

A fatty acid derivative comprising DHA and/or ARA and/or nervonic acid and/or stearic acid may be comprised in the composition of the invention in an amount constituting up to 99.999% of the composition.

In particularly a fatty acid derivative comprising DHA and/or ARA and/or nervonic acid and/or stearic acid, may be comprised in the composition of the invention in an amount of 15 to 350mg/100g dry weight of the composition, more particularly 30mg to 300mg/100g dry weight of the composition.

In an embodiment, the composition according to the present invention comprise a fatty acid derivative comprising DHA and/or ARA and/or nervonic acid and/or stearic acid, in an amount selected from the group consisting of; higher than 15mg/100g, higher than 30 mg/100g, higher than 50 mg/100g, ranging from 30 and 300 mg/100g, ranging from 30 to 200 mg/100g or from 30 to 150 mg/100g, ranging from 50 to 300 mg/100g, ranging from 50 to 200 mg/100g, ranging from 50 to 150 mg/100g dry weight of the composition.

Fatty acid derivatives comprising stearic acid are present in natural sources for example palm oil, tallow, lard, cotton seed oil, peanut oil.

Fatty acid derivatives comprising nervonic acid are resent in natural sources for example the seed oils of Cardamine gracea, Heliphila longifola, Thlaspi perfoliatum, Tropaeolum speciosum, Lunaria biennis, Lunaria annua and Malania oleifera; the moulds Neocallismastix frontalis, Erysiphe graminis and Sphaerotheca humuli; the bacterium Pseudomonas atlantica; the yeast Saccharomyces cerevisiae and the marine diatom Nitzschia cylindrus.

Fatty acid derivatives comprising DHA and/or ARA are present in natural sources such as for example egg, algae, fungus or fish oil.

Oils comprising fatty acid derivatives comprising DHA and/or ARA and generally other polyunsaturated fatty acids (PUFAs), in particular EPA (eicosapentaenoic acid), may be of various origin. Preferably, fatty acid derivatives comprising DHA are provided in the form of a fish oil comprising fatty acid derivatives comprising DHA and/or ARA. Fish oils generally comprise 5wt.% or more, preferably 10wt.% or more of fatty acid derivatives comprising DHA and/or ARA,. Oils comprising substantial amounts of fatty acid derivatives comprising DHA and/or ARA, obtained from algae or microorganisms in general are also available. For example, oils harvested from algae comprising 10wt.% or more, for example 20wt.% or more of fatty acid deivatives, may be used.

If the nutritional composition according to the present invention comprises fatty acid derivatives comprising ARA and DHA. Said ingredients may for example be comprised in the composition of the invention in amounts resulting in a weight ratio of DHA:ARA in the range of 4:1 to 1:4, for example 3:1 to 1:3, for example 2:1 to 1:2, for example 1.5:1 to 1:1.5, in particular 1.1:1 to 1:1.1.

It may also be beneficial if the composition of the invention comprises a mixture of fatty acid derivatives wherein, the mixture is such that the weight ratio of unsaturated to saturated fatty acids and/or fatty acid residues in the composition of the invention is within the range 1:1 to 1:2; 1:1.2 to 1:1.9, 1:1.25 to 1:1.5; 1:3 to 1:4.

Further, when high amounts of fatty acid derivatives comprising DHA and/ or ARA are comprised in the composition of the invention, it may be particularly beneficial if the total amount of fatty acid derivatives comprising saturated long chain fatty acids, in particular C20/24 is increased. These saturated long chain fatty acids may be an important component of myelin enabling it to wrap around and enrobe axons. The weight ratio of DHA and/or AA to these unsaturated long fatty acids in the composition of the invention may for example be within the range 1:1 1:10; 1:2 to 1:9, 1: 3 to 1:4.5, 1:3.5 to 1:4.5.

The nutritional composition according to the present invention comprise ARA.

In one embodiment, the nutritional composition according to the present invention comprise ARA in an amount higher than 30 mg/100g.

In one embodiment, the nutritional composition according to the present invention comprise ARA in an amount higher than 50 mg/100g.

In one embodiment, the nutritional composition according to the present invention comprise ARA in an amount ranging from 30 to 300 mg/100g or from 30 to 200 mg/100g or from 30 to 150 mg/100g.

In one embodiment, the nutritional composition according to the present invention comprise ARA in an amount ranging from 50 to 300 mg/100g or from 50 to 200 mg/100g or from 50 to 150 mg/100g.

ARA is present in natural sources such as for example egg, fungus, algae or fish oil. According to an embodiment, ARA is provided in the form of triglycerides comprising ARA.

Oils comprising ARA and generally other polyunsaturated fatty acids (PUFAs), in particular EPA (eicosapentaenoic acid), may be of various origin. Preferably, the ARA is provided in the form of a fish oil comprising ARA. Fish oils generally comprise 5wt.% or more, preferably 10wt.% or more of ARA. Oils comprising substantial amounts of ARA obtained from algae or microorganisms in general are also available. For example, oils harvested from fungi comprising 10wt.% or more, for example 20wt.% or more of ARA may be used.

ARA may be incorporated in the nutritional compositions of the invention as a single species (as a fatty acid, in the form of a physiologically acceptable salt thereof or in the form of a triglyceride comprising it), as an ingredient consisting of a mixture of different ARA species or by addition of a natural or synthetic ingredient comprising one or more ARA species.

In one embodiment, the nutritional composition of the invention comprises in addition to ARA: Phospholipids (in particular sphingomyelin), minerals (in particular iron, Magnesium, phosphorus, calcium and/or zinc), choline, DHA, Vitamin B12 and/or folic acid.

In another embodiment, the nutritional composition according to the present invention comprise also DHA in addition to a vitamin (for example folic acid and or Vitamin B12).

In one embodiment, the nutritional composition according to the present invention comprise DHA in an amount higher than 30 mg/100g.

In one embodiment, the nutritional composition according to the present invention comprise DHA in an amount higher than 50 mg/100g.

In one embodiment, the nutritional composition according to the present invention comprise DHA in an amount ranging from 30 to 300 mg/100g or from 30 to 200 mg/100g or from 30 to 150 mg/100g.

In one embodiment, the nutritional composition according to the present invention comprise DHA in an amount ranging from 50 to 300 mg/100g or from 50 to 200 mg/100g or from 50 to 150 mg/100g.

The nutritional compositions of the invention comprise DHA.

DHA is present in natural sources such as for example egg, algae or fish oil. According to an embodiment, DHA is provided in the form of triglycerides comprising DHA.

Oils comprising DHA and generally other polyunsaturated fatty acids (PUFAs), in particular EPA (eicosapentaenoic acid), may be of various origin. Preferably, the DHA is provided in the form of a fish oil comprising DHA. Fish oils generally comprise 5wt.% or more, preferably 10wt.% or more of DHA. Oils comprising substantial amounts of DHA obtained from algae or microorganisms in general are also available. For example, oils harvested from algae comprising 10wt.% or more, for example 20wt.% or more of DHA may be used.

DHA may be incorporated in the nutritional compositions of the invention as a single species (as a fatty acid, in the form of a physiologically acceptable salt thereof or in the form of a triglyceride comprising it), as an ingredient consisting of a mixture of different DHA species or by addition of a natural or synthetic ingredient comprising one or more DHA species.

In one embodiment, the nutritional composition according to the present invention comprises arachidonic acid (ARA) in addition to folic acid and DHA.

According to an embodiment, the nutritional composition comprises about the same amounts of DHA and ARA. For example, the weight ratio of DHA:ARA is in the range of 4:1 to 1:4, for example 3:1 to 1:3, for example 2:1 to 1:2, for example 1.5:1 to 1:1.5, in particular 1.1:1 to 1:1.1.

In one embodiment, when high amounts of DHA in the nutritional composition are comprised, the unsaturated long chain fatty acids c20/22/24 are increased.

In one embodiment, the nutritional composition according to the invention comprises folic acid, iron and choline.

In another embodiment, the nutritional composition according to the invention comprises folic acid, iron and DHA.

In a further embodiment, the nutritional composition according to the invention comprises sphingomyelin, iron and folic acid.

In one embodiment, the nutritional composition according to the invention comprises folic acid, DHA and choline.

In another embodiment, the nutritional composition according to the invention comprises folic acid, DHA and iron.

In a further embodiment, the nutritional composition according to the invention comprises sphingomyelin, DHA and folic acid.

In one embodiment, the nutritional composition according to the invention comprises folic acid, choline and DHA.

In another embodiment, the nutritional composition according to the invention comprises folic acid, choline and iron.

In a further embodiment, the nutritional composition according to the invention comprises sphingomyelin, choline and folic acid.

In one embodiment, the nutritional composition according to the invention comprises sphingomyelin, folic acid and DHA.

In another embodiment, the nutritional composition according to the invention comprises sphingomyelin, folic acid and iron.

In a further embodiment, the nutritional composition according to the invention comprises sphingomyelin, folic acid and choline.

In one embodiment, the nutritional composition according to the invention comprises sphingomyelin, folic acid, iron and DHA.

In another embodiment, the nutritional composition according to the invention comprises sphingomyelin, folic acid, choline and iron.

In a further embodiment, the nutritional composition according to the invention comprises sphingomyelin, folic acid, DHA and choline.

In a further embodiment, the nutritional composition according to the invention comprises folic acid, iron, DHA and choline.

In an embodiment, the composition according to the invention comprises a fatty acid derivative, in particular a fatty acid derivative comprising DHA and/or ARA and/or nervonic acid and/or stearic acid, and a vitamin, in particular B12 and/or folic acid, and/or a phospholipid, in particular phosphatidylcholine, and/or phosphatidylserine, and/or phosphatidylinositol, and/or sphingomyelin, and/or a metabolic precursor or metabolite of any of the foregoing, and/or a mineral, in particular iron, and/or zinc, and/or calcium, and/or phosphorus, and/or magnesium and/or choline.

Particularly beneficial concentrations/amounts of said ingredients in said composition may be sphingomyelin in an amount of at least 420mg/kg , phosphatidylcholine in an amount of at least 1000mg/kg, phosphatidylserine in an amount of at least 900mg/kg, phosphatidylinositol in an amount of at least 700mg/kg, folic acid in amount of at least 160mg/kg, vitamin B12 in amount of at least 7mcg/100g, iron in an amount of at least 11.5mg/100g, choline in an amount of at least 140mg/kg, a fatty acid derivative comprising DHA in an amount of at least 89mg/100g, a fatty acid derivative comprising AA in an amount of at least 175mg/100g, zinc in an amount of at least 7mg/100g, calcium in an amount of at least 500mg/100g, phosphorus in an amount of at least 350mg/100g, copper in an amount of at least 600mcg/100g, magnesium in an amount of at least 50mg/100g. Wherein all weights are by dry weight of the composition.

In an embodiment, the composition according to the invention comprises a phospholipid and/or a metabolic precursor or metabolite thereof (for example sphingomyelin, phosphatidylcholine and/or phosphatidylinositol) a fatty acid derivative (for example comprising DHA and/or ARA), vitamin B12 and/or folic acid, , iron, and choline.

In an embodiment, the composition according to the invention comprises a fatty acid derivative comprising DHA and/or ARA, vitamin B12 and/or folic acid, sphingomyelin and iron.

In a more specific embodiment the composition according to the invention comprises a fatty acid derivative comprising DHA in a concentration of 1023mg/kg, ARA in a concentration of 1023mg/kg, vitamin B12 in a concentration of 54mcg/kg, folic acid in a concentration of 1698mcg/kg, sphingomyelin in a concentration of 814mg/kg and iron in a concentration of 67mg/kg.

The composition of the invention may be any type of composition suitable for direct administration to a subject.

In particular the composition will be a synthetic nutritional composition.

In one embodiment, the nutritional composition according to the invention comprises sphingomyelin, folic acid, iron, choline and DHA.

The person skilled in the art would identify appropriate amounts of the above mentioned nutrients, metabolic precursors or metabolites thereof to achieve in the nutritional composition after administration their highest permitted levels.

### Preferred nutritional composition matrix:

The composition according to the invention can be a synthetic nutritional composition. It can be an infant formula, a starter infant formula, a follow-on formula, a growing up milk, a baby food, a preterm formula or a fortifier such as a human milk fortifier, or a supplement. Preferably the composition of the invention is an infant formula, or a fortifier or a supplement intended for the first 4 or 6 months of age.

In one typical embodiment of the present invention, the composition will contain a protein source, a lipid source and a carbohydrate source.

For example such a composition may comprise protein in the range of about 2 to 6 g/100 kcal, lipids in the range of about 1.5 to 3 g/100kcal and/or carbohydrates in the range of about 1.7 to 12 g/100 kcal.

If the composition is liquid, its energy density may be between 60 and 75 kcal/100ml. If the composition is solid, its energy density may be between 60 and 75 kcal/100g.

The type of protein is not believed to be critical to the present invention. Thus, protein sources based on whey, casein and mixtures thereof may be used, for example. As far as whey proteins are concerned, acid whey or sweet whey or mixtures thereof may be used as well as alpha-lactalbumin and beta-lactoglobulin in whatever proportions are desired. The whey protein may be modified sweet whey. Sweet whey is a readily available by-product of cheese making and is frequently used in the manufacture of infant formulas based on cows' milk. However, sweet whey includes a component which is undesirably rich in threonine and poor in tryptophan called caseino-glyco-macropeptide (CGMP). Removal of the CGMP from sweet whey results in a protein with a threonine content closer to that of human milk. This modified sweet whey may then be supplemented with those amino acids in respect of which it has a low content (principally histidine and tryptophan). A process for removing CGMP from sweet whey is described in EP 880902 and an infant formula based on this modified sweet whey is described in WO 01/11990. The proteins may be intact or hydrolysed or a mixture of intact and hydrolysed proteins. It may be desirable to supply partially hydrolysed proteins (degree of hydrolysis between 2 and 20%), for example for subjects believed to be at risk of developing cows' milk allergy. If hydrolysed proteins are required, the hydrolysis process may be carried out as desired and as is known in the art. For example, a whey protein hydrolysate may be prepared by enzymatically hydrolysing the whey fraction in two steps as described in EP 322589. For an extensively hydrolysed protein, the whey proteins may be subjected to triple hydrolysis using Alcalase 2.4L (EC 940459), then Neutrase 0.5L (obtainable from Novo Nordisk Ferment AG) and then pancreatin at 55°C. If the whey fraction used as the starting material is substantially lactose free, it is found that the protein suffers much less lysine blockage during the hydrolysis process. This enables the extent of lysine blockage to be reduced from about 15% by weight of total lysine to less than about 10% by weight of lysine; for example about 7% by weight of lysine which greatly improves the nutritional quality of the protein source.

The compositions of the present invention may contain a carbohydrate source. Any carbohydrate source may be used, such as lactose, saccharose, maltodextrin, starch, honey and mixtures thereof.

The compositions of the present invention may contain a lipid source. The lipid source may be any lipid. Preferred fat sources include milk fat and vegetable oils (including but not limited topalm oil, high oleic sunflower oil and high oleic safflower oil). The essential fatty acids linoleic and α-linolenic acid may also be added. In one embodiment, small amounts of oils containing high quantities of preformed arachidonic acid (AA) and docosahexaenoic acid (DHA) such as fish oils or microbial oils may be added. The lipid source preferably has a ratio of n-6 to n-3 fatty acids of about 5:1 to about 15:1; for example about 8:1 to about 10:1.

The compositions of the present invention may also contain all vitamins and minerals understood to be essential in the daily diet and in nutritionally significant amounts. Minimum requirements have been established for certain vitamins and minerals. Examples of minerals, vitamins and other nutrients optionally present in the infant formula include vitamin A, vitamin B1, vitamin B2, vitamin B6, vitamin B12, vitamin E, vitamin K, vitamin C, vitamin D, folic acid, inositol, niacin, biotin, pantothenic acid, choline, calcium, phosphorous, iodine, iron, magnesium, copper, zinc, manganese, chloride, potassium, sodium, selenium, chromium, molybdenum, taurine, and L-carnitine. Minerals are usually added in salt form. In one embodiment, the composition comprises amounts of Vitamin B12 and/or folic acid. The presence and amounts of specific minerals and other vitamins will vary depending on the numerous factors, such as age weight and condition of the person or animal the composition is administered to.

The compositions may also comprise at least one probiotic bacterial strain. A probiotic is a microbial cell preparation or components of microbial cells with a beneficial effect on the health or well-being of the host. Suitable probiotic bacterial strains include Lactobacillus rhamnosus ATCC 53103 obtainable from Valio Oy of Finland under the trade mark LGG, Lactobacillus rhamnosus CGMCC 1.3724, Lactobacillus paracasei CNCM I-2116, Bifidobacterium lactis CNCM I-3446 sold inter alia by the Christian Hansen company of Denmark under the trade mark Bb12 and Bifidobacterium longum ATCC BAA-999 sold by Morinaga Milk Industry Co. Ltd. of Japan under the trade mark BB536. The amount of probiotic, if present, likewise preferably varies as a function of the age of the person or animal. Generally speaking, the probiotic content may increase with increasing age of the infant for example from 10³ to 10¹² cfu/g formula, more preferably between 10⁴ and 10⁸ cfu/g formula (dry weight).

The compositions may also contain at least one prebiotic in an amount of 0.3 to 10%. A prebiotic is a non-digestible food ingredient that beneficially affects the host by selectively stimulating the growth and/or activity of one or a limited number of bacteria in the colon, and thus improves host health. Such ingredients are non-digestible in the sense that they are not broken down and absorbed in the stomach or small intestine and thus pass intact to the colon where they are selectively fermented by the beneficial bacteria. Examples of prebiotics include certain oligosaccharides.

The compositions may optionally contain other substances which may have a beneficial effect such as nucleotides, nucleosides, and the like.

The compositions, for example an infant formula, for use in the invention may be prepared in any suitable manner. For example, an infant formula may be prepared by blending together the protein source, the carbohydrate source, and the fat source in appropriate proportions. If used, the emulsifiers may be included in the blend. The vitamins and minerals may be added at this point but are usually added later to avoid thermal degradation. Any lipophilic vitamins, emulsifiers and the like may be dissolved into the fat source prior to blending. Water, preferably water which has been subjected to reverse osmosis, may then be mixed in to form a liquid mixture. The liquid mixture may then be thermally treated to reduce bacterial loads. For example, the liquid mixture may be rapidly heated to a temperature in the range of about 80°C to about 110°C for about 5 seconds to about 5 minutes. This may be carried out by steam injection or by heat exchanger; for example a plate heat exchanger. The liquid mixture may then be cooled to about 60°C to about 85°C; for example by flash cooling. The liquid mixture may then be homogenised; for example in two stages at about 7 MPa to about 40 MPa in the first stage and about 2 MPa to about 14 MPa in the second stage. The homogenised mixture may then be further cooled to add any heat sensitive components; such as vitamins and minerals. The pH and solids content of the homogenised mixture is conveniently standardised at this point. The homogenised mixture is transferred to a suitable drying apparatus such as a spray drier or freeze drier and converted to powder. The powder should have a moisture content of less than about 5% by weight. If it is desired to add probiotic(s), they may be cultured according to any suitable method and prepared for addition to the infant formula by freeze-drying or spray-drying for example. Alternatively, bacterial preparations can be bought from specialist suppliers such as Christian Hansen and Morinaga already prepared in a suitable form for addition to food products such as infant formula. Such bacterial preparations may be added to the powdered infant formula by dry mixing.

A vitamin or mixtures thereof may be added at any stage during this procedure, but is preferably added after the heating step.

In one embodiment, the nutritional composition comprises triglycerides with high *sn-*2 palmitate, preferably triglycerides having more than 33% of the palmitic acids in *sn-*2 position.

In some embodiments, palmitic acid comprises from about 15 to about 25%, such as from about 15 to about 20%, of the total fatty acids content of the formula, by weight, and at least from about 30%, for example, from about 35 to about 43% of the total palmitic acid content is in the sn-2 position.

A commercially available composition sold by Lipid Nutrition is Betapol^{™} B-55, which is a triglyceride mixture derived from vegetable oil in which at least 54% of the palmitic acid is in the sn-2 position of the glycerol molecule. In one embodiment, the fat content of the composition of the invention is about 40-50% Betapol^{™} B-55 by weight, for example, from about 43% to about 45% by weight. Those skilled in the art will appreciate that the percentage of the high *sn-*2 fat used and the total amount of *sn-*2 palmitate in the formula may vary, and that a different high sn-2 palmitate oil may be used, without departing from the spirit and scope of the invention.

### Health Effect

The composition of the invention has a positive effect on the myelination trajectory in the brain of the infants or young children who are fed with such compositions.

Such positive effect can comprise the promotion and/or support of an optimal myelination trajectory in the subject fed with the composition, which determines appropriate development of cognitive skills and abilities and learning in the infant or young children. An optimal myelination trajectory may also prevent development of cognitive impairment or delay.

The health effect can be observed after a few days, weeks or months of use of the nutritional composition comprising a vitamin, for example folic acid and/or Vitamin B12.

The effect of the invention can be preventive (for example avoiding a sub-optimal myelination trajectory in the brain) or curative (restoring an optimal myelination trajectory in the brain).

The health effect related to the infant can be measured by various methods as illustrated in the example below.

### Target Infants

In one embodiment of the invention, the target infants or young children show a sub-optimal myelination trajectory in the brain which may result in cognitive deficits, impaired cognitive abilities and/or sub-optimal cognitive development.

In one embodiment, such infants can be preterm or low birth weight infants or infants born small for gestational age.

In another embodiment, the infants or young children are born at term. All infants can benefit from the invention as all infants are or can be susceptible to develop a sub-optimal myelination trajectory in the brain.

In such infants or young children, acquiring a myelination trajectory in the brain that is close to that of breast fed infant (preferably exclusively breast fed infants for the first months of life) is of particular interest. Indeed it provides them with an health status in terms of cognitive abilities which are matching those observed in infants which are breast fed.

In one embodiment, the infants and young children are 0-3 months, 0-6 months, or 0-12 months or 0-36 months of age or 0-60 months of age. It is foreseen that the composition of the invention may be even more beneficial when administered to to infants just after birth (0-4 weeks, 0-8, 0-12, 0-24 weeks) because it is at that time that myelination process has started and significantly develops.

The following examples are presented to illustrate certain embodiments and features of the present invention, but should not be construed as limiting the scope of this invention.

### Intended Feeding regimen

In one embodiment, the composition of the invention is fed to the infant or young children (or intended to be fed or instructed to be fed) for2 to 52 weeks. In one embodiment, it is fed to the infant or young children for 2 to 24 weeks, or 2 to 12 weeks.

In one embodiment, the composition of the invention is fed to the infant or young children (or intended to be fed or instructed to be fed) for 2 to 52 weeks and started shortly after the infants or young children are born or breastfeeding is interrupted. In one embodiment, composition of the invention is fed to the infant or young children for 2 to 24 weeks, or 2 to 12 weeks, and started shortly after the infants or young children are born or breastfeeding is interrupted.

Without wishing to be bound by theory,It is believed that starting early (at birth or close to birth) is preferred to induce the intended effect.

It is expected the health beneficial effect to be more prominent or to established faster when the composition of the invention is used as the exclusive source of nutrition (except if complementing breast feeding). In one embodiment the health effect is observed as long as the composition of the invention is used to cover 50% or more, or 75% or more, of the nutritional needs (e.g. energy needs) of the target infants or young children.

### Experimental section:

### Methods, definitions and materials

### MRI (Magnetic Resonance Imaging):

MRI brain scans of infants and children between 0 and 5 years were acquired using a white matter imaging technique. This technique provides a quantitative measure, the Myelin Water Fraction (MWF), which is a surrogate marker of myelin content. When mapped as a function of time across early childhood, myelination trajectories can be generated.

Infant formula composition: six infant formulas fed to infants participating in study were analyzed for their composition/level of myelin-relevant nutrients.

Composition tested were standard commercial infant formulas of different brands/suppliers and showing variable levels on the nutrients therein contained.

Cognitive abilities: Age-standardized (T) scores of gross motor, visual reception and language (expressive and receptive) derived from the Mullen Scales of Early Learning.

### Clinical study

### Infant Participants

Infants included in this study were drawn from a larger longitudinal study of normal brain and behavioral development: the Brown university Assessment of Myelination and Behavior Across Maturation (BAMBAM). To focus on neurotypical development, children with known potential risk factors for learning, neurologic, or psychiatric disorders were specifically excluded during recruitment and enrollment. Thus, children with *in utero* alcohol or illicit substance exposure, premature (<37 weeks gestation) or multiple birth, fetal ultrasound abnormalities, complicated pregnancy (e.g., preeclampsia), APGAR scores < 8, NICU admission, neurological disorder (e.g., head injury, epilepsy), psychiatric or developmental disorders in the infant, parents or siblings (including maternal depression requiring medication) were excluded. Ongoing screenings, such as the MCAT for autism, or CBCL for behavioral problems, were further used to remove enrolled children with clinically concerning behaviors or overt medical conditions (such as autism spectrum disorders).

A combination of retrospective and prospective data were acquired from parents via detailed medical histories and parental interview on type of infant formula used, percentage of breastfeeding to formula feeding, and length of exclusive breastfeeding. This information was updated at each study visit, which occurred approximately every 6 months for children under 2 years, and yearly for older children. Using this information, children were categorized into one of 2 groups: #1. Exclusively formula-fed; and #2. Exclusively breastfed for at least 90 days (3 months). Children who were fed a combination of breastmilk and formula within 3 months were excluded from our analysis. Infants within the exclusively formula-fed group were further sub-divided based on parental reports of the main infant formula used throughout the first 3 months. Main formula was defined as that given 90% of the time or more (in the case were parents used an alternate brand during vacation, for example).

Using these criteria, 94 exclusively formula-fed infants and young children were selected into group #1. These included 13 children who received formula #2 ; 28 who received formula #5; 8 who received formula #3 ; 39 who received formula #4; 5 who received formula #1 and 1 who received formula #6. A sample of 52 exclusively breastfed infants were also selected and matched to the over formula-fed group with regards to mean age, gestation duration, birth weight, male:female ratio, ethnicity ratio, maternal education, family size, and number of languages spoken in the home (in addition to English). Group demographics are provided in **Table 1.**

**Table 1. Data breakdown for longitudinal and nutritional analysis**

| | Formula 1 | Formula 2 | Formula 5 | Formula 3 | Formula 4 | Formula 6 | Breastfed |
|---|---|---|---|---|---|---|---|
| N_{children} | 5 | 13 | 28 | 8 | 39 | 1 | 52 |
| N_{Measurements} | 11 | 27 | 56 | 14 | 64 | 3 | 106 |

### Imaging Methods and Analysis

Each infant was scanned using the mcDESPOT (multicomponent Driven Equilibrium Single Pulse Observation of T₁ and T₂) white matter imaging technique Deoni et al. (Magn. Reson. Med. 2008, 60:1372-1387) , which provides a quantitative measure of the myelin water fraction (MWF) at each imaging point throughout the brain. All infants were scanned during natural (*i.e. non-sedated*) sleep using acoustically-muffled mcDESPOT imaging protocols. Total imaging times ranged from 19 minutes for the youngest toddlers to 24 minutes for the older 4 year-old children.

All data were acquired on a Siemens 3T Tim Trio scanner equipped with a 12 channel head RF array. To minimize intra-scan motion, children were swaddled with a pediatric MedVac vacuum immobilization bag (CFI Medical Solutions, USA) and foam cushions. Scanner noise was reduced by lessening the peak gradient amplitudes and slew-rates, and using a noise-insulating scanner bore insert (Quiet Barrier HD Composite, UltraBarrier, USA). MiniMuff pediatric ear covers and electrodynamic headphones (MR Confon, Germany) were also used.Children were continuously monitored with a pediatric pulse-oximetry system and infrared camera. All children remained asleep for the duration of the MRI scan and no motion-artifacts were present in the analyzed data.

Following image alignment, non-brain signal removal, and correction for main and transmit magnetic field (B₀ and B₁) inhomogeneities , a three-pool signal model (comprising the myelin-associated water; intra-extra axonal water; and a non-exchanging free-water pool) was fit to the mcDESPOT data to derive voxel-wise MWF maps.

Each child's map was then non-linearly aligned to a study specific template. White matter masks, corresponding to 5 bilateral regions (frontal, temporal, occipital, parietal, and cerebellar WM) as well as the body, genu, and splenium of the corpus callosum were created from common databases, registered to the common template, and superimposed onto each child's MWF map. Mean values for each region were then determined for each child and used for subsequent developmental analysis and trajectory modeling.

### Developmental Differences:

To examine developmental differences between the breastmilk and formula-fed infants, as well as between the different formula-fed infants, a non-linear mixed effects modeling approach was used. Modified Gompertz growth models were fit to groups #1 and #2, and each formula sub-group independently. Each of the four free Gompertz model parameters were then compared between the breast and formula-fed groups using an unpaired t-test, and between the 4 formula sub-groups using an analysis of variance followed by post-hoc Tuckey tests to determine which of the formula groups differed.

### Cognitive Assessments and Analysis

Alongside MR imaging, general cognitive ability and skills were evaluated in each child within 7 days of scanning using the Mullen Scales of Early Learning {MSEL; Mullen:1995vd}. The MSEL provide a broad assessment of behavioral development in the domains of fine and gross motor control, receptive and expressive language, and visual reception. Age-normalized T-scores from these domains can be combined into three composite scores: the early learning composite (ELC, comprising fine motor, visual reception, expressive and receptive language); the non-verbal development quotient (NVDQ, comprising fine motor and visual reception scores); and the verbal development quotient (VDQ, comprising the expressive and receptive language scores).

As with the MWF MRI data, potential group mean differences in ELC, VDQ and NVDQ between the breastmilk and formula-fed infants, as well as between the different formula sub-groups were examined. In addition to mean comparisons, longitudinal changes in these three composite values were investigated using mixed effects modeling assuming a linear trend.

### Example 1

### Nutritional Drivers identification from cross-sectional analyses

From the cohort described above, children up to 5 years of age that were fed different infant formulas during infancy were included in a large correlation analysis to examine the relationship between formula nutrient composition and brain myelination. The 5 most frequently used formulas in that cohort were analyzed for their nutritional composition. A single general linear model (GLM) was constructed that modeled all quantified nutrients, folic acid, and child age. Voxel-wise correlations were calculated with significance defined as p < 0.05 corrected for type 1 error using a cluster based correction approach.

In initial analysis, inclusion of all nutrients resulted in an underpowered model. To reduce the number of variables in the model, we examined the inter-variable correlation. Using a conservative threshold of 0.9, we excluded variables (nutritional components) that were highly correlated with each other across the various formulas. The final model indicated vitamins, especially folic acid and vitamin B12 as nutritional drivers for myelination. Other identified drivers included fatty acid derivative, specifically ARA and/or DHA, phospholipids (especially sphingomyelin, phosphatidylcholine and or phosphatidylinositol), minerals, especially iron, calcium, phosphorus, zinc and Magnesium, and Choline.

For vitamins a significant association with myelination (myelin water fraction) was observed over time in the brain, in particular in the insular cortex.

### Example 2

### Myelination trajectory from longitudinal study

From the available data, analysis of longitudinal development trajectories was performed for two formulas comprising different amount of the nutritional driver identified, i.e. sphingomyelin (composition of such formulas is reported below in table 2):

**Table 2:**

| | **Formula 3** (low folic acid content) | **Formula 1** (high folic acid content) |
|---|---|---|
| folic acid | 107 mcg/100g | 306 mcg/100g |

Results are reported in Fig 1.

The graphs indicate that the higher the folic acid content in the formula, the closer is the myelination trajectory for the infants fed with that formula to that provided by feeding exclusively with human breastmilk.

Based on the results reported in the experimental section, it has been demonstrated that a nutritional composition comprising a vitamin or mixtures thereof determines, in the infant who is fed with such composition, a myelination trajectory in the brain which is optimal (close to that determined via human breast feeding). As above discussed, it is believed that such optimal trajectory in brain myelination corresponds to an effective improvement in the cognitive abilities of the infant, matching abilities of breastfed infants and balancing those lacks in cognitive abilities shown in infants fed with formulas which are low in vitamin acid content.

### Example 3

### Co culture of neurons and OL

Neurons / Oligodendrocytes were cultured as previously described by Charles et al., 2000.

Pregnant female rats of 17 days gestation were killed by cervical dislocation (Rats Wistar) and the foetuses removed from the uterus. The Forebrains were removed and placed in ice-cold medium of Leibovitz (L15) containing 2% of Penicillin-Streptomycin (PS) and 1% of bovine serum albumin (BSA). Forebrains were dissociated by trypsinisation for 20 min at 37ºC (Trypsin EDTA 1X). The reaction was stopped by the addition of Dulbecco's modified Eagle's medium (DMEM) containing DNAase I grade II (0.1 mg/ml) and 10% of foetal calf serum (FCS). Cells were then mechanically dissociated by 3 passages through a 10 ml pipette. Cells were then centrifuged at 180 x g for 10 min at 4°C temperature on a layer of BSA (3.5%) in L15 medium. The supernatant was discarded and the cells of pellet were re-suspended in DMEM containing 10% of FCS. Cells were then centrifuged at 515 x g for 10 min at 4°C. The supernatant was discarded and the cells of pellet were re-suspended in a culture medium consisting of Neurobasal supplemented with 2% of B27, 2 mM of L-glutamine (L Glu), 2% of PS solution, 1 % of FCS and 10 ng/ml of platelet-derived growth factor (PDGF-AA). Viable cells were counted in a Neubauer cytometer using the trypan blue exclusion test. The cells were seeded at a density of 20000 cells/well in 96 well-plates pre-coated with poly-L-lysine and laminin.

The day following seeding (day 1 of culture), cells were incubated with a test compound (**selected from those listed in table 3**), or estradiol. Control cells were not incubated with a test compound or estradiol. Estradiol was used as positive control. Estradiol is known to induce OPC proliferation. The positive effect of estradiol on OL differentiation has also been demonstrated, as has its effect on the early myelination process. The positive effect of estradiol on neurite outgrowth was also published (for review see Alevaro et al., 2010).

The plates were maintained at 37°C in a humidified incubator, in an atmosphere of air (95%)-CO2 (5%). Half of the medium was replaced every other day with fresh medium and test compound or control compound. The test or control compounds were maintained at the defined concentration for the duration of the experiments.

Compounds were tested on 1 culture (6 wells per conditions). Cells were then used on day 12, 18 or 30 of culture to measure one of either proliferation of OPC, differentiation of OPC into OL and early myelination process (myelin wrapping), or maturation of OL (myelin maturation) and mature myelination process (myelin wrapping).

### Proliferation of OPC - Measurement of A2B5 positive cells and total axonal length (NF)

On day 12 of culture, cells were fixed by a cold mixture of absolute ethanol (95%) and pure acetic acid (5%) for 5 min. The cells were then permeabilized and non-specific sites were blocked with a solution of phosphate buffered saline (PBS) containing 0.1% of saponin and 1% FCS for 15 min at room temperature.

Cells were then incubated with Monoclonal Anti-A2B5 conjugated alexa fluor^{®} 488 produced in mouse at dilution of 1/200 in PBS containing 1% FCS, 0.1 % saponin, for 2 h at room temperature and with anti-NF (Neurofilament 200 phosphorylated and non-phosphorylated) produced in rabbit at dilution of 1/500 in PBS containing 1% FCS, 0.1 % saponin for 2 h at room temperature. This antibody was revealed with Alexa Fluor 568 goat anti-rabbit at the dilution of 1/400 in PBS with 1% FCS, 0.1 % saponin, for 1 h at room temperature.

The total number of OPC (number of A2B5 positive cells) was quantified (to evaluate the proliferation), the axonal network was measured (total axonal length (NF)) to assess the effect of the compound on the neuronal network (the quality of the myelination is directly linked to the quality of the axonal network).

### Differentiation of OPC into OL and myelination process (myelin wrapping) - Measurement of number and area of MAG positive cells, overlap MAG/NF wrapping and total axonal length (NF)

On day 18 of culture, cells were fixed by a cold mixture of absolute ethanol (95%) and pure acetic acid (5%) for 5 min. The cells were then permeabilized and non-specific sites were blocked with a solution of phosphate buffered saline (PBS) containing 0.1% of saponin and 1% FCS for 15 min at room temperature.

Cells were then incubated with Monoclonal Anti-MAG produced in mouse at dilution of 1/400 in PBS containing 1% FCS, 0.1 % saponin, and with anti-NF (Neurofilament 200 phosphorylated and non-phosphorylated) produced in rabbit at dilution of 1/500 in PBS containing 1% FCS, 0.1 % saponin for 2 h at room temperature. These antibodies were revealed with CF 488 A goat anti-mouse at the dilution of 1/800 in PBS with 1% FCS, 0.1 % saponin and Alexa Fluor 568 goat anti-rabbit at the dilution of 1/800 in PBS with 1% FCS, 0.1 % saponin, for 1 h at room temperature.

The total number of OL was quantified (number and area of MAG positive cells) (to evaluate the differentiation process), as well as the wrapping of OPC around axons (overlap MAG/NF wrapping) (myelination process). The axonal network was measured (total axonal length (NF) to assess the effect of the compounds on the neuronal network.

### Maturation of OL (myelin maturation) - Measurement of number and area of MBP positive cells, overlap MBP/NF wrapping, and total axonal length (NF)

On day 30 of culture, cells were fixed by a cold mixture of absolute ethanol (95%) and pure acetic acid (5%) for 5 min. The cells were then permeabilized and non-specific sites were blocked with a solution of phosphate buffered saline (PBS) containing 0.1% of saponin and 1% FCS for 15 min at room temperature.

Cells were then incubated with Monoclonal Anti-MBP produced in mouse at dilution of 1/1000 in PBS containing 1% FCS, 0.1 % saponin, and with anti-NF (Neurofilament 200 phosphorylated and non-phosphorylated) produced in rabbit at dilution of 1/500 in PBS containing 1% FCS, 0.1 % saponin for 2 h at room temperature. These antibodies were revealed with CF 488 A goat anti-mouse at the dilution of 1/800 in PBS with 1% FCS, 0.1 % saponin and Alexa Fluor 568 goat anti-rabbit at the dilution of 1/400 in PBS with 1% FCS, 0.1 % saponin, for 1 h at room temperature.

The total number of OL was assessed (number and area of MBP positive cells) (to evaluate the OL maturation) as well as the wrapping of myelin around axon (overlap MBP/NF(wrapping)). The axonal network was measured (Total axonal length (NF)) to assess the effect of the compounds on the neuronal network.

For all measurements, once the culture was done (6 wells per conditions). For each condition tested, 30 pictures (each picture representing a field) per well were taken and analyzed using ImageXpress (Molecular devices) with 20x magnification equipped with LED lamp (excitation 360/480/565 and emission 460/535/620). The 30 pictures were automatically taken and represented 80% of the total surface of the culture well. Results were expressed in terms of cumulated mean length in µm of neurite network, or myelin sheath labeled for a given marker (MAG or MBP) per field. The overlapping area between NF and MAG or MBP was measured to evaluate the wrapping.

To assess OPC population, MAG positive cell population, MBP positive cell population, an automatic counting of number of positive cells per picture (=field) was done. The results were expressed in mean number of positive cells per field.

All the images were taken under the same conditions.

**Table 3**

| **PLATE 1 (A2B5 / NF)** |
|---|
| Control |
| Estradiol (150 nM) |
| DHA (0.15 µM) |
| DHA (1.5 µM) |
| Stearic acid (50 µM) |
| Stearic acid (5 µM) |
| Stearic acid (0.5 µM) |
| B12 (100 nM) |
| B12 (10 nM) |
| B12 (1 nM) |
| Folic acid (250 nM) |
| Folic acid (50 nM) |
| Folic acid (6 nM) |
| Choline (20 µM) |
| Iron (1 µM) |
| Iron (0,1 µM) |
| Zinc (5 µM) |
| Zinc (0,5 µM) |
| Phosphorus (5 mM) |
| Phosphorus (1 mM) |
| Magnesium (25 mM) |
| Copper (0,5 µM) |
| Phosphatidylcholine (100 µM) |
| Phosphatidylinositol (5 µM) |
| Phosphatidylinositol (50 µM) |
| Phosphatidylserine (5 µM) |
| Phosphatidylserine (10 µM) |
| Phosphatidylserine (100 µM) |
| Sphingomyelin (5 µM) |
| Sphingomyelin (25 µM) |
| Ceramide(brain extract):DPPC (1:4) |
| galactoceramides (C18:1/24:1)/(C18:1/18:0):DPPC (1:4) |
| glucoceramides (C18:1/24:1)/(C18:1/18:0):DPPC (1:4) |
| D-erythro-dihydroceramide (C24:1/18:0)/(C18:0/18:1):DPPC (1:4) |
| Ceramide-1-phosphate (C18:1/24:0):DPPC (1:4) |
| GM3:DPPC (1:4) |
| GD3:DPPC (1:4) |

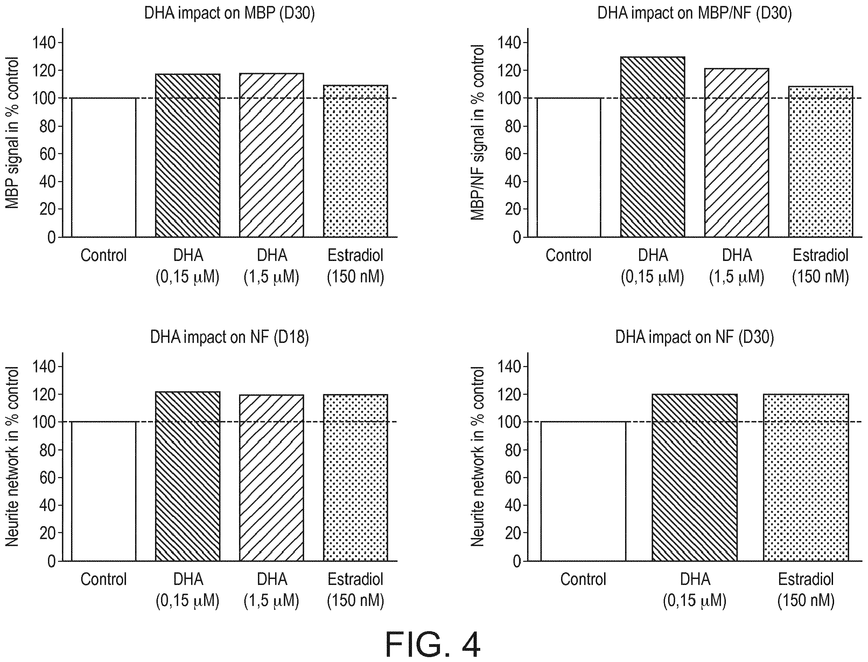

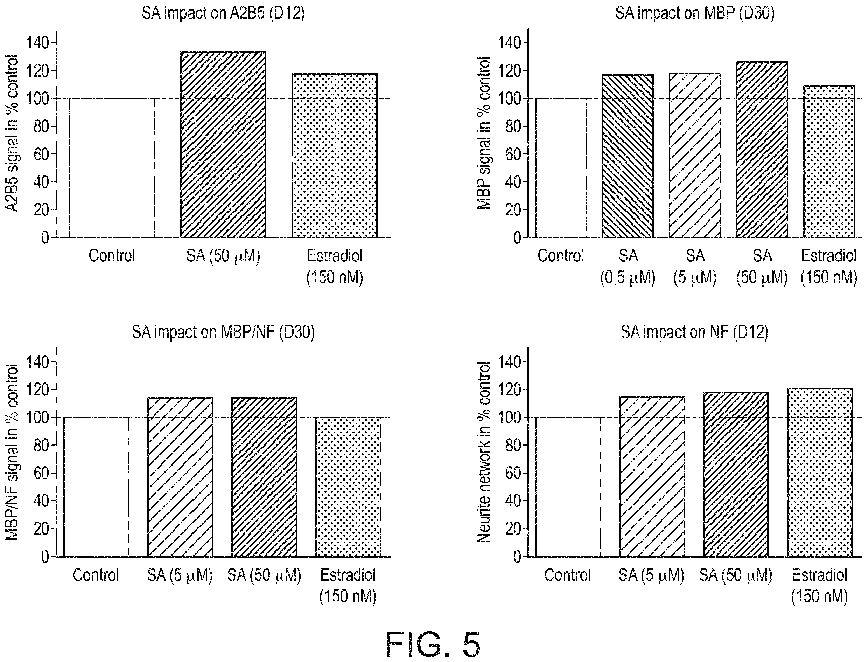

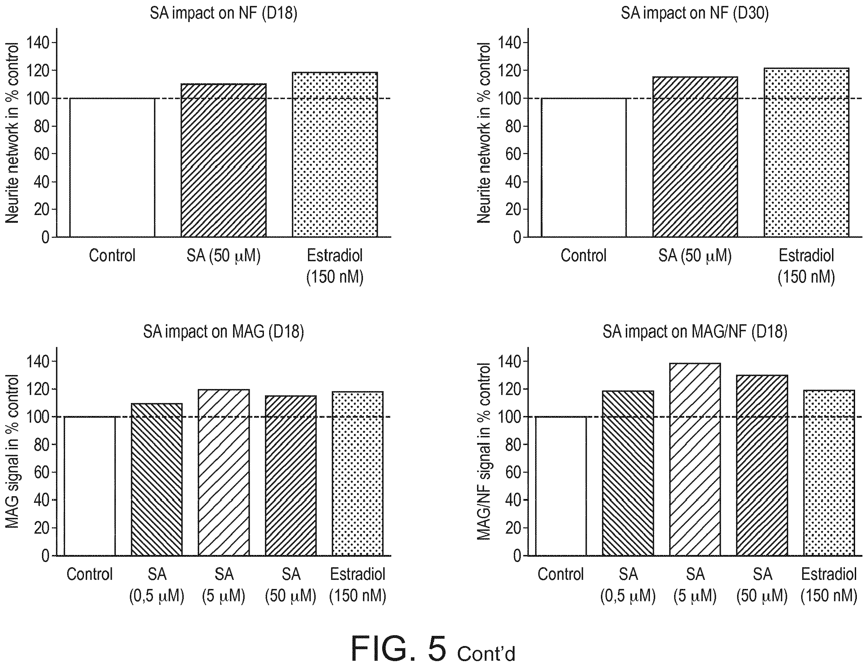

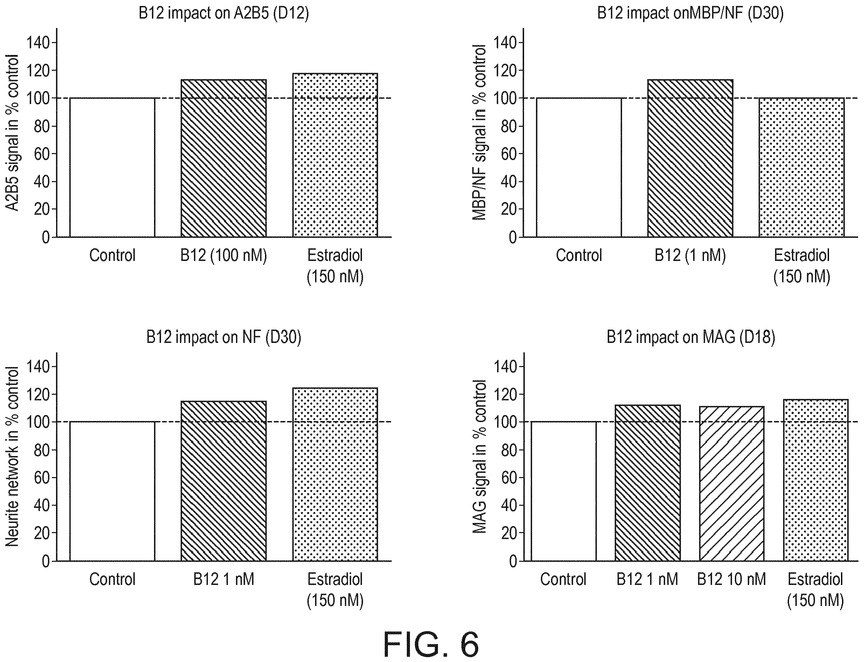

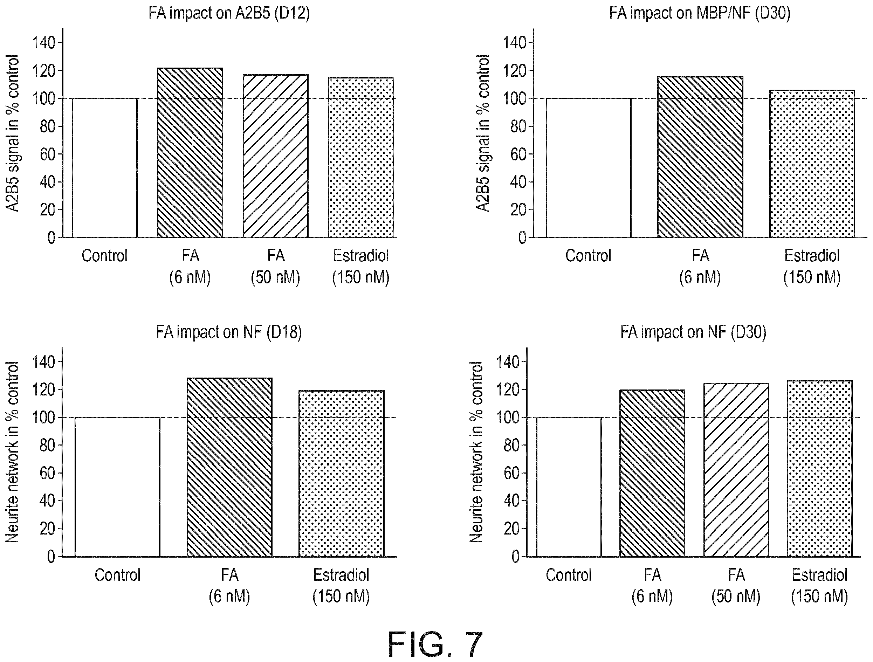

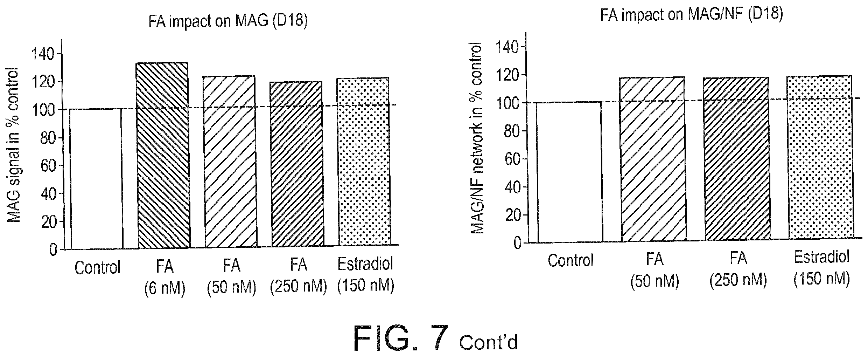

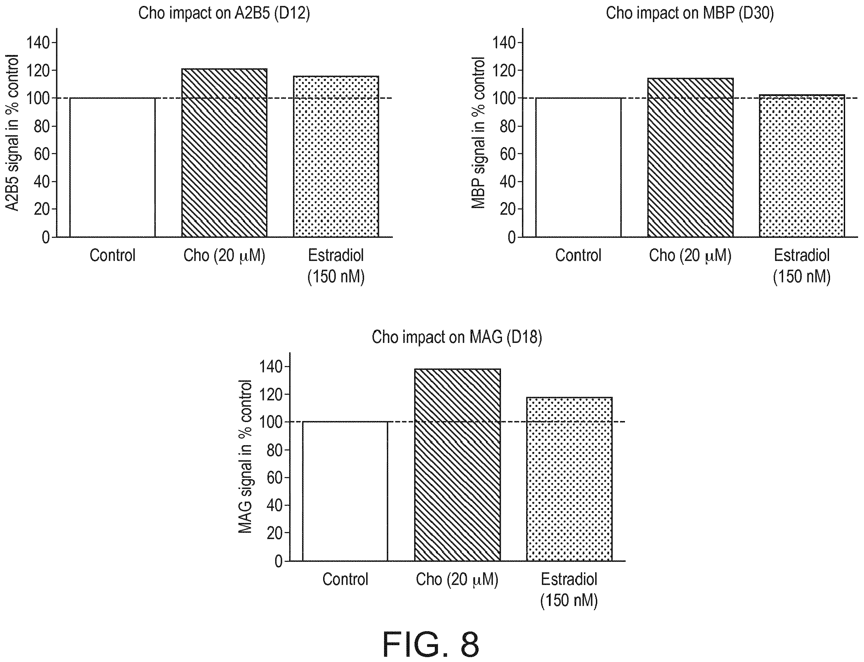

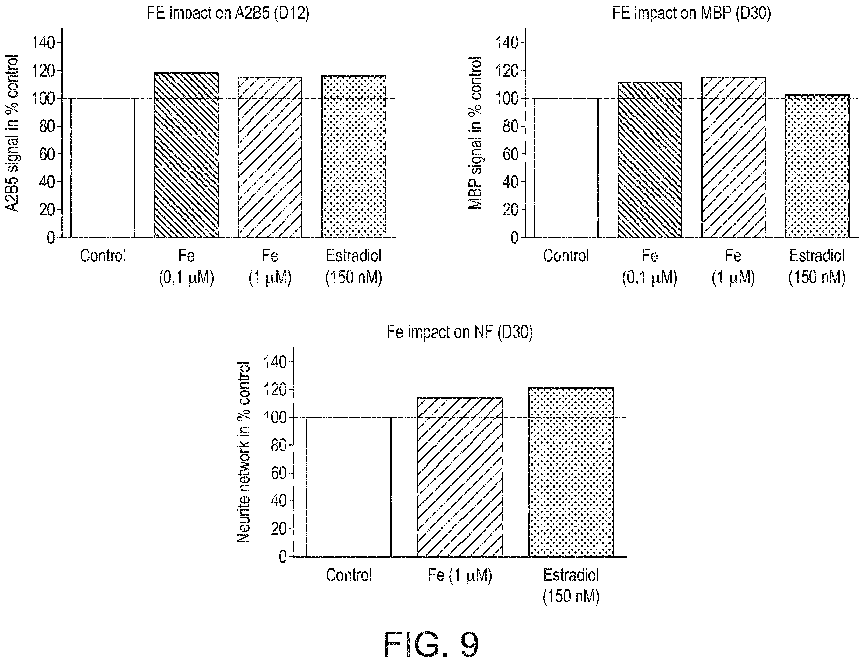

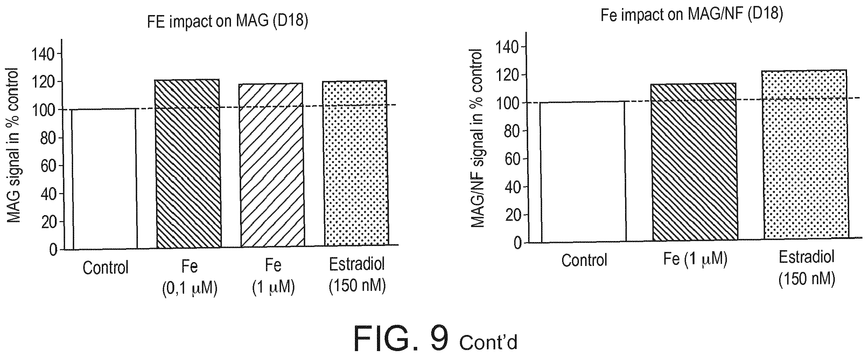

### Results are show in Figures 4 to 24

### Example 4

### Materials and methods

### 1. Feeder layer preparation: Dissociation of neonatal cortices and maintenance of mixed glial cultures

Freshly dissected brains were added to a 37°C water bath for 3 min, then cortices were diced through a P1000 pipette tip to generate smaller fragments. 75 µL of OPC papain solution per brain were added, then tissues were incubated in a 37°C water bath for 20 min. The tissue suspension was then additioned with mixed glial culture in order to allow inactivation of the OPC papain solution.

Tissue were subsequently triturated using a sterile flame-polished glass Pasteur pipette, then 4 mL of mixed glial culture media per brain was added. Cells were centrifuged at 1200 rpm (~300 g) for 5 min, then cells were resuspended in warm mixed glial culture media and plated into PLL-coated flask.

4 hours following plating, a full media change was performed in order to remove much of the debris caused by the trituration, and promote culture viability. After 3 days of culture, a 2/3 media change was performed, and no subsequent medium change was performed. Cells were then maintained in culture until confluency.

### 2. Hippocampal neurons preparation

Hippocampal neurons were isolated from embryonic (E18) pups of Sprague Dawley rats. Briefly, following animal sacrifice, brains were isolated, meninges removed from the medial aspect of the cerebral hemispheres, then hippocampi dissected out and kept at 4°C until process completion.

Tissue were then incubated with 2.5% trypsin for 15 min in a water bath at 37°C, then gently washed and kept in culturing media. Hippocampal dissociation was performed by repeatedly pipetting them up and down with a functionalized sterile Pasteur pipette. Following mechanical dissociation, cells were plated at desired density in neuronal plating medium, let recover for 4 hours, then put in compete neuronal culturing medium.

### 3. Purification of OPCs from mixed glial cultures for establishment of OL/hippocampal neurons o-cultures

On Day 9 of the mixed glial culture, flasks were shaken at 50 rpm for 45 min on an orbital shaker in a 5% CO2 tissue culture incubator. The purpose of this shake was to remove any loosely adherent contaminating cells from the monolayer.

Media was then changed and replaced with 4 mL of fresh mixed glial culture media supplemented with 5 µg/mL insulin. Flasks were then repositioned onto the shaker, equilibrated for approximately 3 hours, then shaken for approximately 16 hours at 220 rpm (overnight).

The next morning, mixed glia culture medium containing microglia and OPCs cells were collected and pre-plated on P100 petri dish (not treated for culture) for 30 minutes in order to purify OPCs cells; microglia cells start immediately to adhere to petri while OPCs cells remained in the surnatant medium.

After 30 minutes of pre-plate, medium was collected and OLs were counted and seeded on hippocampal neurons in a final volume of 1 mL OL media.
A full OL media (minus CNTF) change was performed, then cells were maintained in culture until the appropriate experimental timings.

For maturation experiments, the experimental procedure was as follows:
a. Growth of OPCs on feeder layer of astrocytes for 10 DIV
b. Isolation of OPCs (Day 0)
c. Administration of compounds (Day 3)
d. Quantitative evaluation of maturation at Day 4, 7 and 10.

For myelination experiments, the experimental procedure was as follows:
a. Growth of hippocampal neurons until complete neuronal network maturation (14 DIV)
b. Concomitant growth of OPCs on feeder layer of astrocytes for 10 DIV
c. Isolation of OPCs and coculturing with neurons (Day 14)
d. Administration of compounds (Day 15)
e. Quantitative evaluation of myelination at Day 15 ( 1 day after coculture plating, before compound treatment), 18, 21/23 and 28/29 of coculturing

### 4. Acquisition of images

All cultures at the different experimental time points, were fixed in 4% paraformaldehyde and 4% sucrose at room temperature (RT) for 10 min. Primary and secondary antibodies were applied in GDB buffer (30 mM phosphate buffer, pH 7.4, containing 0.2% gelatin, 0.5% Triton X-100, and 0.8 M NaCl) for 2 h at room temperature. cells were stained with appropriate marker (primary antibody used: Anti-A2B5 antibody (ABCAM cat. ab53521), Rat anti MBP (BIO-RAD cat. aa82-87), Oligodendrocyte Marker O4 Antibody (R&D Systems cat. MAB1326), Anti-βIII Tubulin mAb (Promega cat. G7121); secondary antibody used: Alexa anti rat 555 (Life Tech A-21434), Alexa anti mouse 488 (Life Tech A-11009). Following immunocytochemical staining all images were acquired with Array Scan XTI (ThermoScientific); the objective was 20x at binning 2x2. For each condition and replica well (triplicate) a minimum of 15 images were taken.
For the analysis of all acquired images the HCS Studio Cell Analysis Software was used, in particular the "Scan" application.

### OPC papain solution (made up in MEM)

Papain solution 1.54 mg/mL
L-cysteine 360 µg/mL
DNase I 60 µg/mL

### Mixed glial culture media (made up in DMEM)

FBS 10%
Pen/Strep (0.33% from stock) 33 units/mL Penicillin and 33 µg/mL Streptomycin
GlutaMAX 1%

### OL media

DMEM
100X OL-Supplement
Bovine insulin (from 1 mg/mL stock)
GlutaMAX
Holo-transferrin (from 33 mg/mL stock)
B27 Supplement
FBS
CNTF (from 50 ng/µL stock)

**Results are show in** **Figures 26 to 45**.

The present invention is characterized by the following preferred embodiments:
1. A nutritional composition for infant and/or young children comprising a vitaminor mixtures thereof for promoting and/or supporting an optimal myelination trajectory in the brain.
2. The composition according to item 1 wherein the optimal myelination trajectory in the brain is close to that observed in infants fed exclusively with human breast milk during early months of life.
3. The composition according to anyone of items 1 or 2 wherein it promotes and/or supports an health status characterized by optimal brain and cognitive functions' development and/or prevention of neurocognitive deficits.
4. The composition according to anyone of items 1 to 3 which comprises folic acid in an amount higher than 50 mcg/100g and/or Vitamin B12 in an amount higher than 0.01 mcg/100g.
5. The composition according to anyone of items 1 to 4, which also comprises iron.
6. The composition according to anyone of items 1 to 5, which also comprises DHA.
7. The composition according to anyone of items 1 to 6, which also comprises sphingomyelin.
8. The composition according to anyone of items 1 to 7 which also comprises choline.
9. Use of a composition as described in anyone of items 1 to 8 to promoting and/or supporting an optimal myelination trajectory in the brain of an infant or young children.
10. Use of a composition as described in anyone of items 1 to 8 for preventing a suboptimal myelination trajectory in the brain of an infant or young children .
11. Method for promoting and/or supporting an optimal myelination trajectory in the brain of an infant and/or young children comprising administering to such infant and/or young children a nutritional composition comprising folic acid.
12. Method according to item 11 wherein a composition as described in anyone of items 1 to 8 is administered to infant and/or young children.
13. A nutritional composition comprising:
   Sphingomyelin in an amount higher than 200 mg/kg;
   Iron in an amount higher than 5 mg/100g;
   Choline in an amount higher than 30 mg/100g;
   DHA in an amount higher than 30 mg/100g;
   Folic acid in an amount higher than 50 mcg/100g; and or Vitamin B12 in an amount higher than 0.01 mcg/100g
14. A nutritional composition comprising:
   Sphingomyelin in an amount ranging from 200 mg to 2 g/kg;
   Iron in an amount ranging from 5 to 40 mg/100g;
   Choline in an amount ranging from 30 to 1000 mg/100g;
   DHA in an amount ranging from 30 to 300 mg/100g;
   Folic acid in an amount ranging from 50 to 500 mcg/100g; Vitamin B12 in an amount higher than 0.1 to 10 mcg/100g

## Claims

1. A nutritional composition for infant and/or young children comprising a vitaminor mixtures thereof for promoting and/or supporting an optimal myelination trajectory in the brain.

2. The composition according to claim 1 wherein the optimal myelination trajectory in the brain is close to that observed in infants fed exclusively with human breast milk during early months of life.

3. The composition according to anyone of claims 1 or 2 wherein it promotes and/or supports an health status **characterized by** optimal brain and cognitive functions' development and/or prevention of neurocognitive deficits.

4. The composition according to anyone of claims 1 to 3 which comprises folic acid in an amount higher than 50 mcg/100g and/or Vitamin B12 in an amount higher than 0.01 mcg/100g.

5. The composition according to anyone of claims 1 to 4, which also comprises iron.

6. The composition according to anyone of claims 1 to 5, which also comprises DHA.

7. The composition according to anyone of claims 1 to 6, which also comprises sphingomyelin.

8. The composition according to anyone of claims 1 to 7 which also comprises choline.

9. Use of a composition as described in anyone of claims 1 to 8 to promoting and/or supporting an optimal myelination trajectory in the brain of an infant or young children.

10. Use of a composition as described in anyone of claims 1 to 8 for preventing a suboptimal myelination trajectory in the brain of an infant or young children .

11. Method for promoting and/or supporting an optimal myelination trajectory in the brain of an infant and/or young children comprising administering to such infant and/or young children a nutritional composition comprising folic acid.

12. Method according to claim 11 wherein a composition as described in anyone of claims 1 to 8 is administered to infant and/or young children.

13. A nutritional composition comprising:
Sphingomyelin in an amount higher than 200 mg/kg;
Iron in an amount higher than 5 mg/100g;
Choline in an amount higher than 30 mg/100g;
DHA in an amount higher than 30 mg/100g;
Folic acid in an amount higher than 50 mcg/100g; and or Vitamin B12 in an amount higher than 0.01 mcg/100g

14. A nutritional composition comprising:
Sphingomyelin in an amount ranging from 200 mg to 2 g/kg;
Iron in an amount ranging from 5 to 40 mg/100g;
Choline in an amount ranging from 30 to 1000 mg/100g;
DHA in an amount ranging from 30 to 300 mg/100g;
Folic acid in an amount ranging from 50 to 500 mcg/100g; Vitamin B12 in an amount higher than 0.1 to 10 mcg/100g
